# EUROPEAN PATENT APPLICATION

(11) **EP 2 214 013 A1**
(43) Date of publication of application: **04.08.2010**
(21) Application number: 09001184.2
(22) Date of filing: 28.01.2009
(51) Int. Cl.: G01N 33/50, A61K 38/17

(54) **Methods of treating bone disorders**

(71) Applicant: Universitätsklinikum Freiburg, 79106 Freiburg (DE)
(72) Inventor: Schüle, Roland, 79367 Weisweil (DE); Günther, Thomas, 79104 Freiburg (DE); Yin, Na, 79106 Freiburg (DE)
(74) Representative: Kalhammer, Georg

(57) **Abstract**

The invention relates to the use of a DBC1 inhibitor for the manufacture of a medicament for the treatment and/or prevention of a bone disorder such as osteoporosis. The invention further relates to a method for identifying putative therapeutic agents that may be useful for the treatment of osteoporosis, comprising: (a) providing a candidate compound; (b) assaying for the ability of the candidate compound to inhibit (i) expression of the DBC1 gene in a cell, or (ii) the binding of the DBC1 protein to the osteocalcin promoter.

## Description

### FIELD OF THE INVENTION

The present invention relates to therapeutic and preventive treatments of bone disorders, e.g. osteoporosis. The invention further pertains to methods for identifying putative therapeutic agents that may be useful for the treatment of osteoporosis, to methods of diagnosing a bone disorder and to a transgenic non-human animal, preferably a knockout mouse.

### BACKGROUND OF THE INVENTION

The maintenance of an overall constant bone mass is achieved by a balance between bone matrix deposition and mineralization effected by osteoblasts and resorption caused by osteoclasts (Karsenty and Wagner, 2002). Irregularities in one or more stages of the bone-remodelling cycle (e.g. where the balance between bone formation and resorption is lost) can lead to bone remodelling disorders, or metabolic bone diseases. Examples of such diseases are osteoporosis, Paget's disease and rickets. Some of these diseases are caused by overactivity of one half of the bone-remodelling cycle compared with the other, i.e. by osteoclasts or osteoblasts. In osteoporosis, for example, there is a relative increase in osteoclastic activity which may cause a reduction in bone density and mass. Osteoporosis is the most common of the metabolic bone diseases and may be either a primary disease or may be secondary to another disease or other diseases. On the other hand, osteosclerosis is characterized by abnormal hardening or increased density of bone.

Bone loss is the outcome of imbalanced bone resorption relative to bone formation (Teitelbaum, 2000). Although impaired bone formation contributes to the pathogenesis of osteoporosis, the knowledge of factors involved is limited. Characterization of the molecular mechanisms regulating bone formation is essential to understand the cause of osteoporosis.

There is an ongoing need for novel therapeutic approaches to treat and prevent bone disorders such as osteoporosis.

### SUMMARY OF THE INVENTION

The inventors of this application found that mice lacking the Dbc1 gene exhibit a higher bone formation rate and a higher number of osteoblasts than mice still having the Dbc1 gene. In addition, the inventors surprisingly found that DBC1 promotes the expression of osteocalcin which is a negative regulator of bone growth. The regulation of expression of osteocalcin is effected by binding to a 23 base pair response element identified by the inventors. The present invention is based on these findings.

Accordingly, one aspect of the present invention is the use of a Dbc1 inhibitor for the manufacture of a medicament for the treatment and/or prevention of a bone disorder. The invention also relates to a method of treating a bone disorder which comprises administering to an individual an effective dose of a Dbc1 inhibitor.

Another aspect of the present invention is the use of a Dbc1 nucleic acid for the manufacture of a medicament for the treatment of a bone disorder wherein the Dbc1 nucleic acid is selected from the group consisting of (a) polynucleotides comprising the sequence as shown in SEQ ID NO:1 or 3; (b) polynucleotides comprising a sequence which has an identity of at least 50% to the sequence as shown in SEQ ID NO:1 and/or 3; (c) polynucleotides hybridizing to the sequence as shown in SEQ ID NO:1 and/or 3 under stringent conditions; (d) polynucleotides comprising a sequence which encodes a polypeptide having an amino acid sequence as shown in SEQ ID NO:2 or 4; and (e) polynucleotides comprising a sequence which encodes a polypeptide having an amino acid sequence which has an identity of at least 70% to the amino acid sequence as shown in SEQ ID NO:2 and/or 4.

Yet another aspect of the present invention is a method for identifying putative therapeutic agents that may be useful for the treatment of osteoporosis, comprising:
(a) providing a candidate compound;
(b) assaying for the ability of the candidate compound to inhibit (i) expression of the DBC1 gene in a cell, or (ii) the binding of the DBC1 protein to the osteocalcin promoter.

Yet another aspect of this invention is a method of diagnosing a bone disorder, comprising (a) determining in vitro the level of expression of the DBC1 gene in tissue from an individual; and (b) comparing the level determined in (a) to the level of expression of the DBC1 gene in control tissue; so that if the level determined in (a) is significantly different from that of the control, the individual is diagnosed as exhibiting the bone disorder.

The invention further pertains to a transgenic non-human animal characterized by a decreased level of expression of the Dbc1 gene relative to that of a corresponding wild-type animal.

The present invention further relates to a method for identifying a compound which may be useful as a therapeutic agent for treating osteosclerosis, comprising (a) administering a candidate compound to a transgenic non-human animal of the present invention; (b) determining an activity of the Dbc1 gene or Dbc1 protein; (c) comparing the activity determined in (b) to the activity of the Dbc1 gene or Dbc1 protein in a control animal that has not been contacted with the test compound; and (d) selecting the test compound if the activity measured in (b) is significantly different from that in the control animal.

### BRIEF DESCRIPTION OF THE DRAWINGS

Figure 1 shows detection of Dbc1 (left-hand panel) and DNA (middle panel) in MG-63 cells by immunofluorescence. The right-hand panel shows the merge of both stainings.
Figure 2 shows an analysis of the expression of reporter chain under the control of the human osteocalcin promoter and under the control of a minimal promoter with different elements of this promoter in the human osteosarcoma cell in MG-63 (left-hand panel). Reduction of the expression to the basal level by mutations within the Dbc1 response element (DRE^{mt}) demonstrates the specificity of the effect of Dbc1 on the osteocalcin promoter via this sequence (right-hand panel). The effect of SP1 on the DRE^{wt} promoter-reporter construct was investigated.
Figure 3 shows von Kossa staining of vertebral bodies and tibia in adult wild-type and Dbc1 deficient mice.
Figure 4 shows a comparison of vertebral bodies in adult wild-type and Dbc1 deficient mice The amount of bone substance as a ratio of bone substance to tissue substance (BV/TV), the number of osteoblasts (Nob/Bpm), the bone formation rate (Bfr) and the number of osteoclasts (Noc/Bpm)have been investigated.

### DETAILED DESCRIPTION OF THE INVENTION

The present invention provides methods of treating and/or preventing bone disorders. According to this invention, a Dbc1 inhibitor is administered to a patient suffering from a bone disorder or suspected of being predisposed to this disorder.

A wide variety of bone disorders may be prevented and/or treated in accordance with the present invention, for example all those bone disorders connected with the bone-remodelling cycle. Examples of such diseases include all forms of osteoporosis, osteomalacia, rickets and Paget's disease. Osteoporosis, especially of the post-menopausal, male and steroid-induced types, is of particular note.

"Osteoporosis" is a common bone remodelling disorder characterized by a decrease in bone density of normally mineralized bone, resulting in thinning and increased porosity of bone cortices and trabeculae. The skeletal fragility caused by osteoporosis predisposes sufferers to bone pain and an increased incidence of fractures. Progressive bone loss in this condition may result in a loss of up to 50% of the initial skeletal mass. Primary osteoporosis includes idiopathic osteoporosis which occurs in children or young adults with normal gonadal function, Type I osteoporosis, also described as post-menopausal osteoporosis, and Type II osteoporosis, senile osteoporosis, occurs mainly in those persons older than 70 years of age. Causes of secondary osteoporosis may be endocrine (e.g. glucocorticoid excess, hyperparathyroidism, hypoganodism), drug induced (e.g. corticosteroid, heparin, tobaco) and miscellanous (e.g. chronic renal failure, hepatic disease and malabsorbtion syndrome osteoporosis). Certain factors are well known in the art which may be used to identify those at risk of developing a bone deficit due to bone remodelling disorders like osteoporosis. Important factors include low bone mass, family history, life style, estrogen or androgen deficiency and negative calcium balance. Postmenopausal women are particularly at risk of developing osteoporosis. Hereinafter, references to treatment of bone disorders are intended to include management and/or prophylaxis except where the context demands otherwise.

The methods of the invention may also be used to enhance bone formation in conditions where a bone deficit is caused by factors other than bone remodelling disorders. Such bone deficits include fractures, bone trauma, conditions associated with post-traumatic bone surgery, post-prosthetic joint surgery, post plastic bone surgery, post dental surgery, bone chemotherapy, post dental surgery and bone radiotherapy. Fractures include all types of microscopic and macroscopic fractures. Examples of fractures includes avulsion fracture, comminuted fracture, transverse fracture, oblique fracture, spiral fracture, segmental fracture, displaced fracture, impacted fracture, greenstick fracture, torus fracture, fatigue fracture, intraarticular fracture (epiphyseal fracture), closed fracture (simple fracture), open fracture (compound fracture) and occult fracture.

The methods and uses of this invention may also be used to enhance osteogenesis in a mammal in need thereof. "Osteogenesis," as used herein, refers to proliferation of bone cells and growth of bone tissue (i.e., synthesis and deposit of new bone matrix). In a preferred embodiment, the compounds and compositions of the present invention are used to treat osteoporosis. In another preferred embodiment, the Dbc1 inhibitors are used to increase bone density. In a particularly preferred embodiment, the Dbc1 inhibitors are used to increase bone density and reduce bone loss.

Dbc1 ("deleted in breast cancer 1; 2610301G19Rik) was originally cloned from a fetal brain cDNA library as KIAA1967 (see Nagase et al. (2001) DNA Research 8:319-327). Dbc1 was mapped to chromosome 8p21 in proximity of a region which can be deleted in breast tumors (Hamaguchi et al. (2002) PNAS 99:13647-13652). Dbc1 is expressed ubiquitously (Nagase et al. (2001)). The protein consists of 818 amino acids and shows homology to already known genes. The serines at positions 124, 675, 678 and 681 may be phosphorylated (Beausoleil et al. (2004); Ballif et al. (2004); Molina et al. (2007)). DBC1 comprises an EF domain which mediates the binding of calcium ions. In addition, DBC1 comprises a nuclear localization signal and is characterized by "Leucine Zipper" and "Coiled Coil" motifs which may mediate protein-protein-interactions. The only confirmed interaction partners for DBC1 is are the deacetylase Sirtuin 1 (SIRT1) and the Estrogen Receptor alpha (ERα). The enzymatic activity of SIRT1 is inhibited by DBC1 (Kim et al. (2008); Zhao et al. (2008)). Reduction of the amount of Dbc1 protein in cell culture systems via increased activity of SIRT1 leads to enhanced deacetylation of p53 and, consequently, to a lower sensitivity of the cells to p53-mediated apoptosis (Kim et al. (2008); Zhao et al. (2008)). Cell culture lines which were obtained from non-small-cell lung carcinoma do not express Dbc1. Ectopic expression of Dbc1 in these cell lines leads to a stop of cell proliferation (Izumi et al. (2005)) Trauernicht et al. could show that DBC1 competes with 17-β-estradiol for binding to ERα. Depletion of DBC1 in the cell culture system led to a slight increase of unliganded ERα and to a twofold increase in hormone-independent apoptosis of ERα positive breast cancer cells (Trauernicht et al. (2007))

The nucleotide sequences of human and mouse Dbc1 cDNA are shown in SEQ ID NO:1 and SEQ ID NO:3, respectively. From these sequences also the coding regions (CDS) can be seen. The amino acid sequences of the human and mouse DBC1 protein are shown in SEQ ID NO:2 and 4, respectively.

### Dbc1 Inhibitors

The Dbc1 inhibitor to be used in accordance with this invention is a compound capable of reducing the amount of Dbc1 mRNA or DBC1 protein in a cell and/or inhibiting at least one function of the Dbc1 gene or the DBC1 protein. These functions include (1) the ability of DBC1 to bind to FHL2 protein, (2) the ability of DBC1 to bind to a transcription regulatory element in the 5'-UTR of the osteocalcin gene, and (3) the ability of DBC1 to enhance expression of osteocalcin.

### 1. Compounds capable of inhibiting expression of Dbc1

The Dbc1 inhibitor to be used in accordance with this invention may be a compound capable of inhibiting expression of the Dbc1 gene in a cell. Various methods for inhibiting expression of genes in a cell are known to one of skill in the art. For example, expression of certain genes may be inhibited by using interfering RNA and/or antisense nucleic acids. It is preferred according to the present invention that the Dbc1 inhibitor is selected from siRNA, shRNA, miRNA and antisense nucleic acids.

### a) Interfering RNA

RNA interference (RNAi) is a process by which double-stranded RNA (dsRNA) is used to silence gene expression. While not wanting to be bound by theory, RNAi begins with the cleavage of longer dsRNAs into small interfering RNAs (siRNAs) by an RNaselII-like enzyme, dicer. siRNAs are dsRNAs that are usually about 19 to 28 nucleotides, or 20 to 25 nucleotides, or 21 to 22 nucleotides in length and often contain 2-nucleotide 3' overhangs, and 5' phosphate and 3' hydroxyl termini. One strand of the siRNA is incorporated into a ribonucleoprotein complex known as the RNA-induced silencing complex (RISC). RISC uses this siRNA strand to identify mRNA molecules that are at least partially complementary to the incorporated siRNA strand, and then cleaves these target mRNAs or inhibits their translation. Therefore, the siRNA strand that is incorporated into RISC is known as the guide strand or the antisense strand. The other siRNA strand, known as the passenger strand or the sense strand, is eliminated from the siRNA and is at least partially homologous to the target mRNA. Those of skill in the art will recognize that, in principle, either strand of an siRNA can be incorporated into RISC and function as a guide strand. However, siRNA design (e.g., decreased siRNA duplex stability at the 5' end of the antisense strand) can favor incorporation of the antisense strand into RISC.

RISC-mediated cleavage of mRNAs having a sequence at least partially complementary to the guide strand leads to a decrease in the steady state level of that mRNA and of the corresponding protein encoded by this mRNA. Alternatively, RISC can also decrease expression of the corresponding protein via translational repression without cleavage of the target mRNA. Other RNA molecules and RNA-like molecules can also interact with RISC and silence gene expression. Examples of other RNA molecules that can interact with RISC include short hairpin RNAs (shRNAs), single-stranded siRNAs, microRNAs (miRNAs), and dicer-substrate 27-mer duplexes. The term "siRNA" as used herein refers to a double-stranded interfering RNA unless otherwise noted. Examples of RNA-like molecules that can interact with RISC include RNA- molecules containing one or more chemically modified nucleotides, one or more deoxyribonucleotides, and/or one or more non-phosphodiester linkages. For purposes of the present discussion, all RNA or RNA-like molecules that can interact with RISC and participate in RISC-mediated changes in gene expression will be referred to as "interfering RNAs." siRNAs, shRNAs, miRNAs, and dicer-substrate 27-mer duplexes are, therefore, subsets of "interfering RNAs."

Interfering RNA of embodiments of the invention appear to act in a catalytic manner for cleavage of target mRNA, i.e., interfering RNA is able to effect inhibition of target mRNA in substoichiometric amounts. As compared to antisense therapies, significantly less interfering RNA is required to provide a therapeutic effect under such cleavage conditions.

Nucleic acid sequences cited herein are written in a 5' to 3' direction unless indicated otherwise. The term "nucleic acid," as used herein, refers to either DNA or RNA or a modified form thereof comprising the purine or pyrimidine bases present in DNA (adenine "A," cytosine "C," guanine "G," thymine "T") or in RNA (adenine "A," cytosine "C," guanine "G," uracil "U"). Interfering RNAs provided herein may comprise "T" bases, particularly at 3' ends, even though "T" bases do not naturally occur in RNA. "Nucleic acid" includes the terms "oligonucleotide" and "polynucleotide" and can refer to a single-stranded molecule or a double-stranded molecule. A double-stranded molecule is formed by Watson-Crick base pairing between A and T bases, C and G bases, and between A and U bases. The strands of a double-stranded molecule may have partial, substantial or full complementarity to each other and will form a duplex hybrid, the strength of bonding of which is dependent upon the nature and degree of complementarity of the sequence of bases.

An mRNA sequence is readily deduced from the sequence of the corresponding DNA sequence. For example, SEQ ID NO:1 provides the sense strand sequence of DNA corresponding to the mRNA for Dbc1. The mRNA sequence is identical to the DNA sense strand sequence with the "T" bases replaced with "U" bases. Therefore, the mRNA sequence of Dbc1 is known from SEQ ID NO:1.

Equivalents of the above cited Dbc1 mRNA sequence are alternative splice forms, allelic forms, isozymes, or a cognate thereof. A cognate is a Dbc1 mRNA from another mammalian species that is homologous to SEQ ID NO:1 (an ortholog).

Attenuating expression of an mRNA: The phrase, "attenuating expression of an mRNA," as used herein, means administering or expressing an amount of interfering RNA (e.g., an siRNA) to reduce translation of the target mRNA into protein, either through mRNA cleavage or through direct inhibition of translation. The reduction in expression of the target mRNA or the corresponding protein is commonly referred to as "knock-down" and is reported relative to levels present following administration or expression of a non-targeting control RNA (e.g., a non- targeting control siRNA). Knock-down of expression of an amount including and between 50% and 100% is contemplated by embodiments herein. However, it is not necessary that such knock- down levels be achieved for purposes of the present invention.

Knock-down is commonly assessed by measuring the mRNA levels using quantitative polymerase chain reaction (qPCR) amplification or by measuring protein levels by western blot or enzyme-linked immunosorbent assay (ELISA). Analyzing the protein level provides an assessment of both mRNA cleavage as well as translation inhibition. Further techniques for measuring knock-down include RNA solution hybridization, nuclease protection, northern hybridization, gene expression monitoring with a microarray, antibody binding, radioimmunoassay, and fluorescence activated cell analysis.

Inhibition of Dbc1 may also be determined in vitro by evaluating target mRNA levels or target protein levels in, for example, human osteoblasts following transfection of Dbc1-interfering RNA as described infra.

Interfering RNA: In one embodiment of the invention, interfering RNA (e.g., siRNA) has a sense strand and an antisense strand, and the sense and antisense strands comprise a region of at least near-perfect contiguous complementarity of at least 19 nucleotides. In a further embodiment of the invention, interfering RNA (e.g., siRNA) has a sense strand and an antisense strand, and the antisense strand comprises a region of at least near-perfect contiguous complementarity of at least 19 nucleotides to a target sequence of Dbc1 mRNA, and the sense strand comprises a region of at least near-perfect contiguous identity of at least 19 nucleotides with a target sequence of Dbc1 mRNA, respectively. In a further embodiment of the invention, the interfering RNA comprises a region of at least 13, 14, 15, 16, 17, or 18 contiguous nucleotides having percentages of sequence complementarity to or, having percentages of sequence identity with, the penultimate 13, 14, 15, 16, 17, or 18 nucleotides, respectively, of the 3' end of the corresponding target sequence within an mRNA.

The length of each strand of the interfering RNA comprises 19 to 49 nucleotides, and may comprise a length of 19, 20, 21, 22, 23, 24, 25, 26, 27, 28, 29, 30, 31 , 32, 33, 34, 35, 36, 37, 38, 39, 40, 41 , 42, 43, 44, 45, 46, 47, 48, or 49 nucleotides.

The antisense strand of an siRNA is the active guiding agent of the siRNA in that the antisense strand is incorporated into RISC, thus allowing RISC to identify target mRNAs with at least partial complementary to the antisense siRNA strand for cleavage or translational repression.

In embodiments of the present invention, interfering RNA target sequences (e.g., siRNA target sequences) within a target mRNA sequence are selected using available design tools. Interfering RNAs corresponding to a Dbc1 target sequence are then tested by transfection of cells expressing the target mRNA followed by assessment of knockdown as described above.

Techniques for selecting target sequences for siRNAs are provided by Tuschl, T. et al, "The siRNA User Guide," revised May 6, 2004, available on the Rockefeller University web site; by Technical Bulletin #506, "siRNA Design Guidelines," Ambion Inc. at Ambion's web site; and by other web-based design tools at, for example, the Invitrogen, Dharmacon, Integrated DNA Technologies, Genscript, or Proligo web sites. Basic information can also be obtained from John Matthias et al. Gene silencing by RNAi in mammalian cells in: Current protocols in molecular biology / edited by Frederick M. Ausubel et al. 2003;Chapter 26:Unit 26.2. Initial search parameters can include G/C contents between 35% and 55% and siRNA lengths between 19 and 27 nucleotides. The target sequence may be located in the coding region or in the 5' or 3' untranslated regions of the mRNA.

An embodiment of a 21-nucleotide DNA target sequence for human DBC1 mRNA is
TAGTCATGCAAGCTGGTAACA (SEQ ID NO:5)

Each "N" residue can be any nucleotide (A, C, G, U, T) or modified nucleotide. The 3' end can have a number of "N" residues between and including 1, 2, 3, 4, 5, and 6. The "N" residues on either strand can be the same residue (e.g., UU, AA, CC, GG, or TT) or they can be different (e.g., AC, AG, AU, CA, CG, CU, GA, GC, GU, UA, UC, or UG). The 3 ' overhangs can be the same or they can be different. In one embodiment, both strands have a 3 'UU overhang.

As cited in the examples above, one of skill in the art is able to use the target sequence information provided in Table 1 to design interfering RNAs having a length shorter or longer than the sequences provided in the table and by referring to the sequence position in SEQ ID NO:1 and adding or deleting nucleotides complementary or near complementary to SEQ ID NO: 1 respectively.

The target RNA cleavage reaction guided by siRNAs and other forms of interfering RNA is highly sequence specific. In general, siRNA containing a sense nucleotide strand identical in sequence to a portion of the target mRNA and an antisense nucleotide strand exactly complementary to a portion of the target mRNA are siRNA embodiments for inhibition of mRNAs cited herein. However, 100% sequence complementarity between the antisense siRNA strand and the target mRNA, or between the antisense siRNA strand and the sense siRNA strand, is not required to practice the present invention. Thus, for example, the invention allows for sequence variations that might be expected due to genetic mutation, strain polymorphism, or evolutionary divergence.

In one embodiment of the invention, the antisense strand of the siRNA has at least near-perfect contiguous complementarity of at least 19 nucleotides with the target mRNA. "Near-perfect," as used herein, means the antisense strand of the siRNA is "substantially complementary to," and the sense strand of the siRNA is "substantially identical to" at least a portion of the target mRNA. "Identity," as known by one of ordinary skill in the art, is the degree of sequence relatedness between nucleotide sequences as determined by matching the order and identity of nucleotides between the sequences. In one embodiment, the antisense strand of an siRNA having 80% and between 80% up to 100% complementarity, for example, 85%, 90% or 95% complementarity, to the target mRNA sequence are considered near-perfect complementarity and may be used in the present invention. "Perfect" contiguous complementarity is standard Watson- Crick base pairing of adjacent base pairs. "At least near-perfect" contiguous complementarity includes "perfect" complementarity as used herein. Computer methods for determining identity or complementarity are designed to identify the greatest degree of matching of nucleotide sequences, for example, BLASTN (Altschul, S.F., et al. (1990) J. Mol. Biol. 215:403-410).

The term "percent identity" describes the percentage of contiguous nucleotides in a first nucleic acid molecule that is the same as in a set of contiguous nucleotides of the same length in a second nucleic acid molecule. The term "percent complementarity" describes the percentage of contiguous nucleotides in a first nucleic acid molecule that can base pair in the Watson-Crick sense with a set of contiguous nucleotides in a second nucleic acid molecule.

The relationship between a target mRNA (sense strand) and one strand of an siRNA (the sense strand) is that of identity. The sense strand of an siRNA is also called a passenger strand, if present. The relationship between a target mRNA (sense strand) and the other strand of an siRNA (the antisense strand) is that of complementarity. The antisense strand of an siRNA is also called a guide strand.

The penultimate base in a nucleic acid sequence that is written in a 5' to 3' direction is the next to the last base, i.e., the base next to the 3' base. The penultimate 13 bases of a nucleic acid sequence written in a 5' to 3' direction are the last 13 bases of a sequence next to the 3' base and not including the 3' base. Similarly, the penultimate 14, 15, 16, 17, or 18 bases of a nucleic acid sequence written in a 5' to 3' direction are the last 14, 15, 16, 17, or 18 bases of a sequence, respectively, next to the 3' base and not including the 3' base.

The phrase "a region of at least 13 contiguous nucleotides having at least 90% sequence complementarity to, or at least 90% sequence identity with, the penultimate 13 nucleotides of the 3' end of an mRNA corresponding to any one of (a sequence identifier)" allows a one nucleotide substitution. Two nucleotide substitutions (i.e., 11/13 = 85% identity/complementarity) are not included in such a phrase.

In one embodiment of the invention, the region of contiguous nucleotides is a region of at least 14 contiguous nucleotides having at least 85% sequence complementarity to, or at least 85% sequence identity with, the penultimate 14 nucleotides of the 3' end of an mRNA corresponding to the sequence identified by each sequence identifier. Two nucleotide substitutions (i.e., 12/14 = 86% identity/complementarity) are included in such a phrase.

In a further embodiment of the invention, the region of contiguous nucleotides is a region of at least 15, 16, 17, or 18 contiguous nucleotides having at least 80% sequence complementarity to, or at least 80% sequence identity with, the penultimate 14 nucleotides of the 3' end of an mRNA corresponding to the sequence of the sequence identifier. Three nucleotide substitutions are included in such a phrase.

The target sequence in the mRNAs corresponding to SEQ ID NO:1 may be in the 5' or 3' untranslated regions of the mRNA as well as in the coding region of the mRNA.

One or both of the strands of double-stranded interfering RNA may have a 3' overhang of from 1 to 6 nucleotides, which may be ribonucleotides or deoxyribonucleotides or a mixture thereof. The nucleotides of the overhang are not base-paired. In one embodiment of the invention, the interfering RNA comprises a 3' overhang of TT or UU. In another embodiment of the invention, the interfering RNA comprises at least one blunt end. The termini usually have a 5' phosphate group or a 3' hydroxyl group. In other embodiments, the antisense strand has a 5' phosphate group, and the sense strand has a 5' hydroxyl group. In still other embodiments, the termini are further modified by covalent addition of other molecules or functional groups.

The sense and antisense strands of the double-stranded siRNA may be in a duplex formation of two single strands as described above or may be a single molecule where the regions of complementarity are base-paired and are covalently linked by a hairpin loop so as to form a single strand. It is believed that the hairpin is cleaved intracellularly by a protein termed dicer to form an interfering RNA of two individual base-paired RNA molecules.

Interfering RNAs may differ from naturally-occurring RNA by the addition, deletion, substitution or modification of one or more nucleotides. Non-nucleotide material may be bound to the interfering RNA, either at the 5' end, the 3' end, or internally. Such modifications are commonly designed to increase the nuclease resistance of the interfering RNAs, to improve cellular uptake, to enhance cellular targeting, to assist in tracing the interfering RNA, to further improve stability, or to reduce the potential for activation of the interferon pathway. For example, interfering RNAs may comprise a purine nucleotide at the ends of overhangs. Conjugation of cholesterol to the 3' end of the sense strand of an siRNA molecule by means of a pyrrolidine linker, for example, also provides stability to an siRNA.

Further modifications include a 3' terminal biotin molecule, a peptide known to have cell-penetrating properties, a nanoparticle, a peptidomimetic, a fluorescent dye, or a dendrimer, for example.

Nucleotides may be modified on their base portion, on their sugar portion, or on the phosphate portion of the molecule and function in embodiments of the present invention. Modifications include substitutions with alkyl, alkoxy, amino, deaza, halo, hydroxyl, thiol groups, or a combination thereof, for example. Nucleotides may be substituted with analogs with greater stability such as replacing a ribonucleotide with a deoxyribonucleotide, or having sugar modifications such as 2' OH groups replaced by T amino groups, 2' O-methyl groups, T methoxyethyl groups, or a 2'-O, 4'-C methylene bridge, for example. Examples of a purine or pyrimidine analog of nucleotides include a xanthine, a hypoxanthine, an azapurine, a methylthioadenine, 7-deaza-adenosine and O- and N-modified nucleotides. The phosphate group of the nucleotide may be modified by substituting one or more of the oxygens of the phosphate group with nitrogen or with sulfur (phosphorothioates). Modifications are useful, for example, to enhance function, to improve stability or permeability, or to direct localization or targeting.

There may be a region or regions of the antisense interfering RNA strand that is (are) not complementary to a portion of SEQ ID NO: 1. Non-complementary regions may be at the 3', 5' or both ends of a complementary region or between two complementary regions.

Interfering RNAs may be generated exogenously by chemical synthesis, by in vitro transcription, or by cleavage of longer double-stranded RNA with dicer or another appropriate nuclease with similar activity. Chemically synthesized interfering RNAs, produced from protected ribonucleoside phosphoramidites using a conventional DNA/RNA synthesizer, may be obtained from commercial suppliers such as Ambion Inc. (Austin, TX), Invitrogen (Carlsbad, CA), or Dharmacon (Lafayette, CO). Interfering RNAs are purified by extraction with a solvent or resin, precipitation, electrophoresis, chromatography, or a combination thereof, for example. Alternatively, interfering RNA may be used with little if any purification to avoid losses due to sample processing.

Interfering RNAs can also be expressed endogenously from plasmid or viral expression vectors or from minimal expression cassettes, for example, PCR generated fragments comprising one or more promoters and an appropriate template or templates for the interfering RNA. Examples of commercially available plasmid-based expression vectors for shRNA include members of the pSilencer series (Ambion, Austin, TX) and pCpG-siRNA (InvivoGen, San Diego, CA). Viral vectors for expression of interfering RNA may be derived from a variety of viruses including adenovirus, adeno-associated virus, lentivirus (e.g., HW, FIV, and EIAV), and herpes virus. Examples of commercially available viral vectors for shRNA expression include pSilencer adeno (Ambion, Austin, TX) and pLenti6/BLOCK-iT™-DEST (Invitrogen, Carlsbad, CA).

Selection of viral vectors, methods for expressing the interfering RNA from the vector and methods of delivering the viral vector are within the ordinary skill of one in the art. Examples of kits for production of PCR-generated shRNA expression cassettes include, Silencer Express (Ambion, Austin, TX) and siXpress (Minis, Madison, WI). A first interfering RNA may be administered via in vivo expression from a first expression vector capable of expressing the first interfering RNA and a second interfering RNA may be administered via in vivo expression from a second expression vector capable of expressing the second interfering RNA, or both interfering RNAs may be administered via in vivo expression from a single expression vector capable of expressing both interfering RNAs.

Interfering RNAs may be expressed from a variety of eukaryotic promoters known to those of ordinary skill in the art, including pol III promoters, such as the U6 or HI promoters, or pol II promoters, such as the cytomegalovirus promoter. Those of skill in the art will recognize that these promoters can also be adapted to allow inducible expression of the interfering RNA.

Hybridization under Physiological Conditions: In certain embodiments of the present invention, an antisense strand of an interfering RNA hybridizes with an mRNA in vivo as part of the RISC complex.

"Hybridization" refers to a process in which single-stranded nucleic acids with complementary or near-complementary base sequences interact to form hydrogen-bonded complexes called hybrids. Hybridization reactions are sensitive and selective. In vitro, the specificity of hybridization (i.e., stringency) is controlled by the concentrations of salt or formamide in prehybridization and hybridization solutions, for example, and by the hybridization temperature; such procedures are well known in the art. In particular, stringency is increased by reducing the concentration of salt, increasing the concentration of formamide, or raising the hybridization temperature.

For example, high stringency conditions could occur at about 50% formamide at 37°C to 42 °C. Reduced stringency conditions could occur at about 35% to 25% formamide at 30°C to 35°C. Examples of stringency conditions for hybridization are provided in Sambrook, J., 1989, Molecular Cloning: A Laboratory Manual, Cold Spring Harbor Laboratory Press, Cold Spring Harbor, N. Y. Further examples of stringent hybridization conditions include 400 mM NaCl, 40 mM PIPES pH 6.4, 1 mM EDTA, 50°C or 70°C for 12-16 hours followed by washing, or hybridization at 70°C in IxSSC or 50°C in IxSSC, 50% formamide followed by washing at 70°C in 0.3xSSC, or hybridization at 70°C in 4xSSC or 50°C in 4xSSC, 50% formamide followed by washing at 67°C in IxSSC. The temperature for hybridization is about 5-10°C less than the melting temperature (Tₘ) of the hybrid where Tₘ, is determined for hybrids between 19 and 49 base pairs in length using the following calculation: Tₘ,°C = 81.5 + 16.6(log₁₀[Na⁺]) + 0.41 (% G+C) - (600/N) where N is the number of bases in the hybrid, and [Na⁺] is the concentration of sodium ions in the hybridization buffer.

The above-described in vitro hybridization assay provides a method of predicting whether binding between a candidate siRNA and a target will have specificity. However, in the context of the RISC complex, specific cleavage of a target can also occur with an antisense strand that does not demonstrate high stringency for hybridization in vitro.

Single-stranded interfering RNA: As cited above, interfering RNAs ultimately function as single strands. Single-stranded (ss) interfering RNA has been found to effect mRNA silencing, albeit less efficiently than double-stranded siRNA. Therefore, embodiments of the present invention also provide for administration of a ss interfering RNA that hybridizes under physiological conditions to a portion of SEQ ID NO:1 and has a region of at least near-perfect contiguous complementarity of at least 19 nucleotides with the hybridizing portion of SEQ ID NO:1, respectively. The ss interfering RNA has a length of 19 to 49 nucleotides as for the ds siRNA cited above. The ss interfering RNA has a 5' phosphate or is phosphorylated in situ or in vivo at the 5' position. The term "5' phosphorylated" is used to describe, for example, polynucleotides or oligonucleotides having a phosphate group attached via ester linkage to the C5 hydroxyl of the sugar (e.g., ribose, deoxyribose, or an analog of same) at the 5' end of the polynucleotide or oligonucleotide.

SS interfering RNAs are synthesized chemically or by in vitro transcription or expressed endogenously from vectors or expression cassettes as for ds interfering RNAs. 5' Phosphate groups may be added via a kinase, or a 5' phosphate may be the result of nuclease cleavage of an RNA. Delivery is as for ds interfering RNAs. In one embodiment, ss interfering RNAs having protected ends and nuclease resistant modifications are administered for silencing. SS interfering RNAs may be dried for storage or dissolved in an aqueous solution. The solution may contain buffers or salts to inhibit annealing or for stabilization.

Hairpin interfering RNA: A hairpin interfering RNA is a single molecule (e.g., a single oligonucleotide chain) that comprises both the sense and antisense strands of an interfering RNA in a stem-loop or hairpin structure (e.g., a shRNA). For example, shRNAs can be expressed from DNA vectors in which the DNA oligonucleotides encoding a sense interfering RNA strand are linked to the DNA oligonucleotides encoding the reverse complementary antisense interfering RNA strand by a short spacer. If needed for the chosen expression vector, 3' terminal T's and nucleotides forming restriction sites may be added. The resulting RNA transcript folds back onto itself to form a stem-loop structure.

Mode of administration: Interfering RNA may be delivered via aerosol, buccal, dermal, intradermal, inhaling, intramuscular, intranasal, intraocular, intrapulmonary, intravenous, intraperitoneal, nasal, ocular, oral, otic, parenteral, patch, subcutaneous, sublingual, topical, or transdermal administration, for example.

Formulations and Dosage: Pharmaceutical formulations comprise interfering RNAs, or salts thereof, of the invention up to 99% by weight mixed with a physiologically acceptable carrier medium such as water, buffer, saline, glycine, hyaluronic acid, mannitol, and the like.

Interfering RNAs of the present invention are administered as solutions, suspensions, or emulsions. The following are examples of possible formulations embodied by this invention.

### b) Antisense nucleic acids

Antisense nucleic acids may be used to inhibit the expression of the Dbc1 gene in a cell. Methods and techniques for designing and preparing antisense nucleic acids are known per se. The skilled person can provide suitable antisense nucleic acids on the basis of information derived from the nucleotide sequence of Dbc1. Suitable techniques are described, e.g., in Asubel et al. ed., chapter 26 (1994-2008).

### 2. Compounds capable of inhibiting the ability of Dbc1 to bind to a transcription regulatory element in the 5'-UTR of the osteocalcin gene.

In another embodiment the invention, the Dbc1 inhibitor is capable of inhibiting the binding of DBC1 protein to the osteocalcin promoter. Such inhibitors include antibodies specifically binding to the Dbc1 protein. The antibodies are preferably monoclonal antibodies. Techniques for generating monoclonal antibodies specifically binding to specific proteins are known to those of skill in the art (see, e.g., Harlow and Lane, Antibodies: A Laboratory Manual, Cold Spring Harbor 1988. Cold Spring Harbor Laboratory; or ULLMANN'S Biotechnology and Biochemical Engineering, pp 443-455, Wiley-VCH 2007). It is also possible to use nucleic acids which hybridize with the target sequence within the osteocalcin promoter and thus prevent binding of Dbc1 protein to the osteocalcin promoter.

The human OSTEOCALCIN promoter has the nucleic acid sequence as shown in SEQ ID NO:12 (GenBank sequence DQ007079, position 3235-4096):

One of skill in the art will appreciate that the Dbc1 inhibitors can be used alone or in combination with other compounds and therapeutic regimens to induce osteogenesis. For example, the inhibitor may be administered in conjunction with bone morphogenetic proteins or anti-resorptive medications that affect the bone remodeling cycle. Suitable bone morphogenetic proteins include, for example, BMP-2, BMP-4, and BMP-7. Suitable anti-resorptive medications include, for example, bisphosphonates such as, for example, alendronate sodium and risedronate sodium; hormones, such as, for example, calcitonin and estrogens, and selective estrogen receptor modulators, such as, for example, raloxifene.

An effective amount of the inhibitor will be determined by the existence, nature, and extent of any adverse side-effects that accompany the administration of the composition; the LD₅₀ of the composition; and the side-effects of the composition at various concentrations. Typically, the amount of the composition administered will range from about 0.01 to about 20 mg per kg, more typically about 0.05 to about 15 mg per kg, even more typically about 0.1 to about 10 mg per kg body weight.

The inhibitor can be administered, for example, by intravenous infusion, orally, intraperitoneally, or subcutaneously. Oral administration is the preferred method of administration. The formulations of compounds can be presented in unit-dose or multi-dose sealed containers, such as ampoules and vials.

The Dbc1 inhibitors are typically formulated with a pharmaceutically acceptable carrier before administration to an individual or subject. Pharmaceutically acceptable carriers are determined, in part, by the particular composition being administered, as well as by the particular method used to administer the composition. Accordingly, there are a wide variety of suitable formulations of pharmaceutical compositions of the present invention (see, e.g., Remington's Pharmaceutical Sciences, 17th ed., 1989).

Formulations suitable for oral administration can consist of (a) liquid solutions, such as an effective amount of the compound of Formula I suspended in diluents, such as water, saline or PEG 400; (b) capsules, sachets or tablets, each containing a predetermined amount of the active ingredient, as liquids, solids, granules or gelatin; (c) suspensions in an appropriate liquid; and (d) suitable emulsions. Tablet forms can include one or more of the following: lactose, sucrose, mannitol, sorbitol, calcium phosphates, corn starch, potato starch, microcrystalline cellulose, gelatin, colloidal silicon dioxide, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise the active ingredient in a flavor, e.g., sucrose, as well as pastilles comprising the active ingredient in an inert base, such as gelatin and glycerin or sucrose and acacia emulsions, gels, and the like containing, in addition to the active ingredient, carriers known in the art.

The compositions of the present invention may be in formulations suitable for other routes of administration, such as, for example, intravenous infusion, intraperitoneally, or subcutaneously. The formulations include, for example, aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. Injection solutions and suspensions can be prepared from sterile powders, granules, and tablets.

The dose administered to a patient, in the context of the present invention should be sufficient to effect a beneficial therapeutic response in the patient over time. For example, if the compositions of the present invention are administered to treat or prevent osteoporosis, the dose administered to the patient should be sufficient to prevent, retard, or reverse decreases in bone density. The dose will be determined by the efficacy of the particular composition employed and the condition of the patient, as well as the body weight or surface area of the patient to be treated. The size of the dose also will be determined by the existence, nature, and extent of any adverse side-effects that accompany the administration of a particular composition in a particular patient.

The Dbc1 inhibitors can also be used to induce or enhance osteogenesis in vitro by contacting mammalian cells, e.g. osteoblast cells, with the Dbc1 inhibitor. Suitable cells and cell lines include 7F2 (CRL-12557), MC3T3 E1 (CRL-2593), MG-63 (CRL-1427), primary osteoblasts, Ros17, ST2, U-2 OS (HTB-96), UMR-106 (CRL-1661) etc.

Methods of culturing mammalian cells are known to the skilled person. Suitable techniques are for example summarized in Bonifacino et al. ed., chapter 1 (1998-2008).

### Detection of osteogenesis

After administration of the compositions of the present invention in vivo or in vitro, induction of osteogenesis can be detected by detecting expression of osteoblast-specific proteins, detecting expression of bone-specific transcription factors, and detecting changes in bone density. Osteoblast-specific proteins include, for example, alkaline phosphatase (ALP), collagen type I, osteocalcin, and osteoponin (see, e.g., Olsen et al., Annu. Rev. Cell. Dev. Biol. 16:191 (2000)). Typically, expression of alkaline phosphatase is detected as an indicator of osteogenesis. Bone specific transcription factors include, for example, Cbfa1/Runx2, gsc, Dlx1, Dlx5, Msx1, Cart1, Hoxa1, Hoxa2, Hoxa3, Hoxb1, rae28, Twist, AP-2, Mf1, Pax1, Pax3, Pax9, TBX3, TBX4, TBX5, and Brachyury (see, e.g., Olsen et al, 2000 supra). Typically, expression of Cbfa1/Runx2 is detected as an indicator of osteogenesis.

### 1. Detection of Osteoblast-Specific Proteins

Expression of osteoblast-specific proteins may be detected by measuring the level of the osteoblast-specific protein or mRNA. The level of particular osteoblast-specific proteins can conveniently be measured using immunoassays such as immunohistochemical staining, western blotting, ELISA and the like with an antibody that selectively binds to the particular osteoblast specific proteins or a fragment thereof. Detection of the protein using protein-specific antibodies in immunoassays is known to those of skill in the art (see, e.g., Harlow & Lane, Antibodies: A Laboratory Manual (1988), Coligan, Current Protocols in Immunology (1991); Goding, Monoclonal Antibodies: Principles and Practice (2d ed. 1986); and Kohler & Milstein, Nature 256:495-497 (1975). For measurement of mRNA, amplification, e.g., PCR, LCR, or hybridization assays, e.g., northern hybridization, RNAse protection, dot blotting, are preferred. The level of protein or mRNA is detected, for example, using directly or indirectly labeled detection agents, e.g., fluorescently or radioactively labeled nucleic acids, radioactively or enzymatically labeled antibodies. These assays are well-known to those of skill in the art and described in, e.g., Ausubel, et al. ed. CURRENT PROTOCOLS IN MOLECULAR BIOLOGY (2001).

Typically, expression of the osteoblast specific-protein, alkaline phosphatase, is used to detect differentiated osteoblasts. Expression of alkaline phosphatase (ALP) is correlated with osteogenesis. ALP hydrolyzes inorganic pyrophosphates to phosphates and promotes the formation of hydroxyapatite crystals in bone matrix. Deactivating mutations of ALP cause osteomalacia, characterized by poorly mineralized bones and frequent bone factures, indicating that ALP plays a significant role in bone formation (see, e.g., Hessle et al., Proc. Natl. Acad. Sci. USA. 99:9445(2002)). ALP is a highly active and stable enzyme, making direct assays of its enzymatic activity convenient. In addition, direct histochemical staining of cells can conveniently be used to detect ALP.

### 2. Detection of Bone-Specific Transcription Factors

Expression of bone-specific transcription factors can be detected using reporter gene assays. These assays are well known to those of skill in the art and are described in, e.g., Ausebel et al., supra. Expression of the bone specific transcription factor Cbfa1/Runx2 is typically used to detect osteogenesis. Cbfa1/Runx2 plays an essential role in osteoblast differentiation transgenic mice lacking the Cbfa1/Runx2 gene die shortly after birth due to loss in bone formation (see, e.g., Ducy et al., Cell 89:747 (1997) and Komori et al., Cell 89:755 (1997)).

Reporter genes such as, for example, chloramphenicol acetyltransferase, firefly luciferase, bacterial luciferase, or .beta.-galactosidase can be used in the reporter gene assays. The reporter construct is typically transiently or stably transfected into a cell. The promoter region of the relevant gene is typically amplified by PCR appropriate primers. The resulting PCR product is inserted into a suitable cloning vector, amplified and sequenced. The resulting plasmid is digested with appropriate restriction enzymes and the resulting fragment is inserted into a vector comprising a reporter gene.

### 3. Detection of Bone Density

To assess the effect of a compound on bone density, a baseline measurement of bone density in an individual who will receive treatment may taken. Bone density is periodically measured at suitable intervals during and after administration of the compound. Methods and devices for measuring bone density are well known in the art and are described in, e.g., U.S. Pat. Nos. 6,436,042; 6,405,068; 6,320,931; 6,302,582; 6,246,745; 6,230,036; 6,213,934; 6,102,567; 6,058,157; 5,898,753; 5,891,033; 5,852,647; 5,817,020; 5,782,763; 5,778,045; 5,749,363; 5,745,544; 5,715,820; 5,712,892; 5,572,998; and 5,480,439.

### Dbc1 nucleic acids

The invention further relates to the use of a Dbc1 nucleic acid for the manufacture of a medicament for the treatment of a bone disorder wherein the DBC1 nucleic acid is selected from the group consisting of (a) polynucleotides comprising the sequence as shown in SEQ ID NO:1 or 3; (b) polynucleotides comprising a sequence which has an identity of at least 50%, preferably at least 60%, more preferably at least 70%, more preferably at least 80%, most preferably at least 90% to the sequence as shown in SEQ ID NO:1 and/or 3; (c) polynucleotides hybridizing to the sequence as shown in SEQ ID NO:1 and/or 3 under stringent conditions; (d) polynucleotides comprising a sequence which encodes a polypeptide having an amino acid sequence as shown in SEQ ID NO:2 or 4; and (e) polynucleotides comprising a sequence which encodes a polypeptide having an amino acid sequence which has an identity of at least 70% to the amino acid sequence as shown in SEQ ID NO:2 and/or 4. The stringent conditions referred to herein include, for example, hybridization at 20 mM to 40 mM NaCl at a temperature of about 50°C to about 70°C, preferably at about 60°C to 65°C.

The bone disorder may be characterized by an increased bone mass relative to that of non-diseased bone (e.g. osteosclerosis). In another embodiment, the disorder is characterized by a deacreased bone mass relative to non-diseased bone (e.g. osteoporosis).

### Screening methods

The invention further relates to a method for identifying putative therapeutic agents that may be useful for the treatment of a bone disorder (e.g. osteoporosis), comprising:
(a) providing a candidate compound;
(b) assaying for the ability of the candidate compound to inhibit (i) expression of the DBC1 gene in a cell, or (ii) the binding of the DBC1 protein to the osteocalcin promoter.

The ability of the candidate compound to inhibit expression of the DBC1 gene in a cell can be determined according the techniques generally known in the art. For example, step (b) may comprise: (i) contacting the candidate compound with at least one cell in vitro ; (ii) determining the level of expression of the DBC1 gene in the cell(s); (iii) comparing the level of DBC1 expression determined in (ii) to the level of DBC1 expression in at least one control cell that has not been contacted with the candidate compound; and (iv) selecting the candidate compound if the level of DBC1 expression determined in (ii) is lower than that in the at least one control cell. Methods to determine the expression of a gene in a cell are known to the skilled person.

The skilled person can also determine the ability of the candidate compound to inhibit the binding of the DBC1 protein to the osteocalcin promoter using techniques that are known per se. These include competition assay, gel shift assay and the like. For example, step (b) may comprise: (i) contacting DBC1 protein with a nucleic acid comprising the osteocalcin promoter so as to allow formation of a complex between the DBC1 protein and the nucleic acid ; (ii) determining the amount of complex formed in (i); (iii) adding the candidate compound to the complex formed between DBC1 protein and the osteocalcin promoter; (iv) determining the amount of complex still present after (iii);and (v) selecting the candidate compound if the amount of complex determined in (iv) is lower than that determined in (ii).

Alternatively, step (b) may comprise: (i) contacting DBC1 protein with a nucleic acid comprising the osteocalcin promoter in the absence of the candidate compound so as to allow formation of a complex between the DBC1 protein and the nucleic acid; (ii) determining the amount of complex formed in (i); (iii) contacting DBC1 protein with a nucleic acid comprising the osteocalcin promoter in the presence of the candidate compound under the same conditions as in (i); (iv) determining the amount of complex formed in (iii);and (v) selecting the candidate compound if the amount of complex determined in (iv) is lower than that determined in (ii).

The invention further relates to a method for the preparation of a compound that is useful in the treatment of a bone disorder, said method comprising: (a) identifying a compound by the method described hereinabove; and (b) synthesizing the compound by chemical syntesis.

### Methods of diagnosis

The invention further relates to a method of diagnosing a bone disorder, comprising (a) determining in vitro the level of expression of the DBC1 gene in tissue from an individual; and (b) comparing the level determined in (a) to the level of expression of the DBC1 gene in control tissue; so that if the level determined in (a) is significantly different from that of the control, the individual is diagnosed as exhibiting the bone disorder.

The level of Dbc1 expression may be determined as described supra, e. g. , by measuring the amount/concentration of mRNA in the cells or by measuring the amount/concentration of Dbc1 protein present in the cells. The determination can be carried out using a tissue sample derived from an individual. Preferably, the tissue sample comprises bone material, more preferably the tissue sample comprises osteoblasts. The tissue sample may be obtained through biopsy from an individual. In a specific embodiment, osteoblasts are enriched after having obtained the biopsy and prior to determining the level of expression of the Dbc1 gene.

Independently of step (a), one can determine in vitro the level of expression of the Dbc1 gene in tissue or cells from a healthy control individual. Preferably, the type of tissue or cells to be examined as control is the same type of tissue or cells as that used in step (a). It is important that the control individual does not suffer from a bone disease, e. g. , osteoporosis. From the amount of Dbc1 mRNA or protein one can derive the concentration of Dbc1 mRNA or protein, given as g/ (number of cells), mol/ (number of cells), g/ (g cell mass), mol/ (g cell mass), or the like.

Usually, the individual is diagnosed as exhibiting a bone disease characterized by increased bone density (e.g. osteosclerosis) or as being at risk of developing the disease, if the level determined in step (a) is lower than that of the control. In specific embodiments, the individual is diagnosed as exhibiting the bone disease or as being at risk of developing the disease, if the level of Dbc1 expression (e. g. , the amount or concentration of the Dbc1 mRNA or the amount or concentration of the Dbc1 protein) determined in (a) is less than 90% or less than 80% or less than 70% or less than 60% or less than 50% of the level of Dbc1 expression of the control. Diseases characterized by increased bone density include Melorheostosis, Osteopetrosis and Osteopoikilosis.

In another embodiment the individual is diagnosed as exhibiting a bone disease characterized by decreased bone density (e.g. osteoporosis) or as being at risk of developing the disease, if the level determined in step (a) is higher than that of the control. In specific embodiments, the individual is diagnosed as exhibiting the bone disease or as being at risk of developing the disease, if the level of Dbc1 expression (e. g., the amount or concentration of the Dbc1 mRNA or the amount or concentration of the Dbc1 protein) determined in (a) is higher than 110% or higher than 120% or higher than 130% or higher than 140% or higher than 150% of the level of Dbc1 expression of the control. Diseases characterized by increased bone density have been described supra. Most preferably, the bone disease to be diagnosed is osteoporosis.

### Transgenic animals

The invention further relates to transgenic non-human animals.

The term "animal" is used herein to include all vertebrate animals, except humans. It also includes an individual animal in all stages of development, including embryonic and fetal stages. A "transgenic animal" is any animal containing one or more cells bearing genetic information altered or received, directly or indirectly, by deliberate genetic manipulation at the subcellular level, such as by targeted recombination or microinjection or infection with recombinant virus. The term "transgenic animal" is not meant to encompass classical cross-breeding or in vitro fertilization, but rather is meant to encompass animals in which one or more cells are altered by or receive a recombinant DNA molecule. This molecule may be specifically targeted to defined genetic locus, be randomly integrated within a chromosome, or it may be extrachromosomally replicating DNA. The term "germ cell line transgenic animal" refers to a transgenic animal in which the genetic alteration or genetic information was introduced into a germ line cell, thereby conferring the ability to transfer the genetic information to offspring. If such offspring in fact, possess some or all of that alteration or genetic information, then they, too, are transgenic animals.

The alteration or genetic information may be foreign to the species of animal to which the recipient belongs, or foreign only to the particular individual recipient, or may be genetic information already possessed by the recipient. In the last case, the altered or introduced gene may be expressed differently than the native gene.

Techniques are available to inactivate or alter any genetic region to a mutation desired by using targeted homologous recombination to insert specific changes into chromosomal alleles. However, in comparison with homologous extrachromosomal recombination, which occurs at a frequency approaching 100%, homologous plasmid-chromosome recombination was originally reported to only be detected at frequencies between 10⁻⁶ and 10⁻³. Nonhomologous plasmid-chromosome interactions are more frequent occurring at levels 10⁵ -fold to 10² -fold greater than comparable homologous insertion.

To overcome this low proportion of targeted recombination in murine ES cells, various strategies have been developed to detect or select rare homologous recombinants. One approach for detecting homologous alteration events uses the polymerase chain reaction (PCR) to screen pools of transformant cells for homologous insertion, followed by screening of individual clones. Alternatively, a positive genetic selection approach has been developed in which a marker gene is constructed which will only be active if homologous insertion occurs, allowing these recombinants to be selected directly. One of the most powerful approaches developed for selecting homologous recombinants is the positive-negative selection (PNS) method developed for genes for which no direct selection of the alteration exists. The PNS method is more efficient for targeting genes which are not expressed at high levels because the marker gene has its own promoter. Non-homologous recombinants are selected against by using the Herpes Simplex virus thymidine kinase (HSV-TK) gene and selecting against its nonhomologous insertion with effective herpes drugs such as gancyclovir (GANC) or (1-(2-deoxy-2-fluoro-B-D arabinofluranosyl)5-iodouracil, (FIAU). By this counter selection, the fraction of homologous recombinants in the surviving transformants can be increased.

As used herein, a "targeted gene" or "knock-out" is a DNA sequence introduced into the germline or a non-human animal by way of human intervention, including but not limited to, the methods described herein. The targeted genes of the invention include DNA sequences which are designed to specifically alter cognate endogenous alleles.

Methods of use for the transgenic mice of the invention are also provided herein. Such mice may be used to advantage to identify agents which inhibit or modify osteogenesis. For example, therapeutic agents for the treatment or prevention of osteosclerosis may be screened in studies using the knockout mice of the present invention. Such assays will not only facilitate the identification of agents which regulate osteosclerosis, they should also be illustrative of the underlying biochemical mechanisms which underlie the disorder.

The level of expression of the Dbc1 gene in the transgenic non- human animal may be less than 90%, preferably less than 75%, more preferably less than 50% and still more preferably less than 25% of the level of Dbc1 expression in the non- modified non-human animal. The amount or concentration ofmRNA or Dbc1 protein in bone tissue may be a measure for the level of expression of the Dbc1 gene.

Most preferably, the non-human animal completely lacks expression of functional Dbc1 protein. In that embodiment, the Dbc1 gene may have been deleted or rendered non-functional.

It is also preferred that the non-human animal is a non-human mammal, more preferably the non-human animal is a rodent, most preferably, the non-human animal is a mouse. r The non-human animal may have been manipulated to be missing all or essentially all of an activity of one or more specific gene/allele products. In a preferred embodiment, the non-human animal has been manipulated so as not to express functional Dbc1 protein.

In addition, according to the invention cells, preferably animal cells, and more preferably mammalian cells that have been manipulated to be missing all or essentially all of an activity of the Dbc1 protein, may be useful as osteoporosis model systems. The cells may be human or non-human cells. Preferably, the cells are bone cells, e. g., osteoblasts.

The invention provides methods of making and using these cells and non-human animals.

The knockout animals and/or corresponding cells of the present invention can be manipulated to be incapable of expressing a functional protein from one or more specified alleles and/or genes by any means known in the art. For example, a knockout animal and/or corresponding cell can be manipulated to comprise a disruption in an endogenous allele and/or gene, e. g. , the Dbc1 gene, thereby preventing the expression of the corresponding functional protein. Alternatively, a knockout animal and/or corresponding cell can be manipulated to comprise a dominant mutant allele. Fhl2 knockout mice have been described in Kong et al. 2001, Circulation 103,2731-2738.

In yet another embodiment, a knockout animal and/or corresponding cell can be treated with one or more antisense nucleic acids for one or more specific gene (s) thereby inhibiting the expression of the specific gene (s). In still another embodiment, the gene (s) encoding the specific functional protein (s) can be constructed such that the expression of the protein (s) is under the control of an inducible promoter, and this expression is conditionally repressed. In still another embodiment, the cell and/or nun-human animal is treated with/administered inhibitory compound (s) that prevent the expression and/or activity of the Dbc1 protein.

The invention includes the use of a non-human knockout animal that comprises a homozygous disruption in its endogenous Dbc1 gene as an osteosclerosis model.

The non-human animal preferably shows increased bone formation rate relative to that of the corresponding wild-type animal. The bone formation rate may be increased by at least 5%, preferably at least 10%, most preferably at least 15%, compared to the corresponding wild-type animal. The bone formation rate may be determined as described in WO 2005/001482 or as described supra.

The non-human animal preferably shows an increase of the amount of the calcified extracellular matrix. This increase may concern the vertebral bodies of the non-human animal. The amount of the calcified extracellular matrix may be increased by at least 10%, preferably by at least 20%, more preferably by at least 25%, most preferably by at least about 30%, compared to the amount of the calcified extracellular matrix in a corresponding wild-type animal. The increase can be present in male or in female animals.

According to the invention also a cell from the transgenic non-human animal of the invention may be useful. Preferably the cell is from the knockout mouse and has the same genetic background as the knockout mouse.

The non-human animal described supra can be used as an in vivo osteosclerosis model. The non-human animals offer the unique possibility to test the effect of pharmacological substances on the activity of osteoblasts and therefore their efficacy in osteosclerosis treatment. The invention relates to the use of an Dbc1-deficient non-human animal for the development of a medicament for the treatment of bone disease, e. g. , osteosclerosis.

The invention further relates to a method for identifying a putative agent for reducing bone mass, said method comprising: (a) administering a candidate compound to a transgenic non-human animal described hereinabove; (b) comparing the bone formation rate or the bone density of said transgenic animal to the bone formation rate or the bone density in a control animal that has not been contacted with the candidate compound; and (c) selecting the candidate compound if the bone formation rate or the bone density in the transgenic animal is significantly lower than that in the control animal. The control animal is another transgenic animal having substantially the same genetic background as the transgenic animal treated with the candidate compound. Methods of determining bone density and bone formation rate are known to those of ordinary skill.

The various embodiments of the invention described herein may be combined.

The following nucleic acid and amino acid sequences are shown in the sequence listing:

| **SEQ ID NO:** | **Sequence description** | **GenBank acc. #** |
|---|---|---|
| 1 | hDbc1 cDNA | NM_021174 |
| 2 | hDbc1 protein | NM_021174 |
| 3 | mDbc1 cDNA | BC021475 |
| 4 | mDbc1 protein | BC021475 |
| 5 | DNA target for siRNA | |
| 6 | mOsteocalcin cDNA | NM_001032298 |
| 7 | mOsteocalcin preproprotein | NM_001032298 |
| 8 | mOsteocalcin mature peptide | NP_001027469 |
| 9 | hOsteocalcin cDNA | NM_199173 |
| 10 | hOsteocalcin preproprotein | NM_199173 |
| 11 | hOsteocalcin mature peptide | NP_954642 |
| 12 | hOsteocalcin promoter | DQ007079 |

The following examples illustrate the invention without being limiting:

### Example 1: Dbc1 is a nuclear protein

### Immunofluorescence:

A polyclonal antibody α-DBC1 was generated against recombinant full-length DBC1 in rabbit. This antibody reacts specifically with human and mouse DBC1 in Western blots and immunohistochemistry. For endogenous DBC1 staining MG-63 cells were seeded on coverslips. Cells were washed twice the next day with phosphate-buffered saline (PBS), fixed with 4% parafomaldehyde/PBS for 10 min, permeabilized with 0.2% Triton X-100/PBS and blocked in 0.2% gelatine/PBS overnight. Staining with the polyclonal α-DBC1 antibody (diluted 1:1000 in 0.2% gelatine/PBS) was followed by washing five times with PBS. The secondary Alexa 488-labelled antibody (Molecular Probes) was used at a dilution of 1:2000 in 0.2% gelatine/PBS followed by washing five times with PBS. Nuclei were stained with DAPI (Vectashield mounting medium for fluorescence with DAPI, Vector).

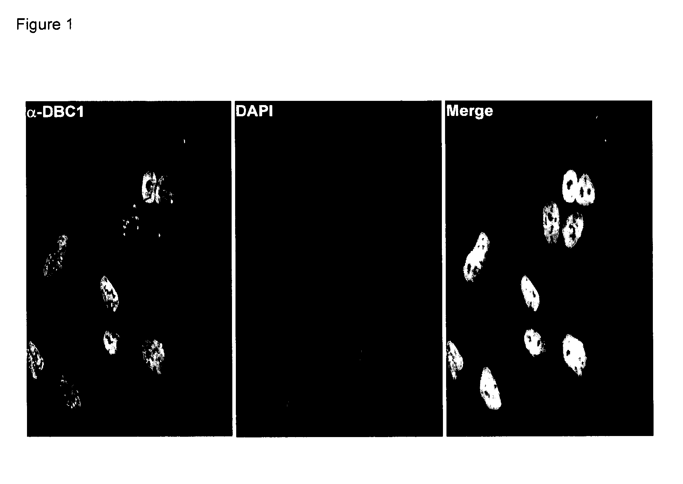

Dbc1 was detected via indirect immunofluorescence (left-hand panel of Fig. 1). The DNA of the cell nuclei was visualized in parallel using DAPI (middle panel of Fig. 1). The right-hand panel of Fig. 1 shows the merge of both stainings. As can be seen, Dbc1 is a nuclear protein.

### Example 2: Dbc1 directs expression of the osteoblast marker osteocalcin

### Transient transfections:

MG-63 cells were cultured in EMEM supplemented with 10% fetal calf serum Transient transfection assays were carried out in 24 well plates (2.5 × 104 cells per well). Cells were transfected with Effectene (Qiagen). The total amount of transfected DNA was kept constant (375 ng/well) by pCMX and pUC18. Reporter plasmids (125 ng), 100 ng and 25ng of pCMX-Flag-DBC1, 12ng to 100ng of CMV SP1 were transfected per well. Luciferase activity was assayed as described (Günther et al., 2005). All experiments were repeated at least five times in duplicate.

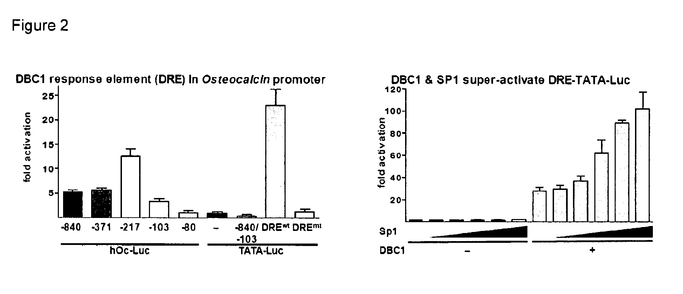

Analysis of the expression of reporter chain under the control of the human osteocalcin promoter and under the control of a minimal promoter with different elements of this promoter in the human osteosarcoma cell in MG-63 is shown in the left-hand panel of Fig. 2. The promoting influence of Dbc1 on expression is mediated by a cis-regulatory element of the promoter between position -103 and -80. A fragment comprising 23 base pairs from this region (DREwt) is sufficient to obtain this effect. Reduction of the expression to the basal level by mutations within the Dbc1 response element (DRE^{mt}) demonstrates the specificity of the effect of Dbc1 on the osteocalcin promoter via this sequence (right-hand panel of Fig. 2). The effect of SP1 on the DRE^{wt} promoter-reporter construct is minimal. Cotransfection of Dbc1 in increasing amounts, however, leads to a synergistic and concentration-dependent increase in expression.

### Example 3: Dbc1-deficient mice exhibit an increase in bone substance

### Generation of Dbc1-deficient mice:

We have used the embryonic stem cell clone XN876 from the International Gene Trap Consortium (Nord et al. 2006) for blastocyst injection to generate chimeras by standard procedures (Asubel et al., chapter 23). Integration of a gene trap vector into intron 7 led to the introduction of a STOP codon after the 31st amino acid. Chimeras were bred with wild-type C57BL/6 mice for germ line transmission. Heterozygous mice were backcrossed to C57BU6 mice to generate congenic mice. Deficiency for Dbc1 protein in homozygous knockout mice was verified by Western blot and immunohistochemistry.

### Bone histomorphometry:

Histomorphometry was performed on age- and sex-matched incipient congenic mice (n>4). For histological analysis, bone substance was stained by the von Kossa method. Quantitative histomorphometry was performed on toluidine blue-stained, undecalcified, proximal tibial and vertebral sections. Experiments were performed in a blinded fashion. Analysis of bone volume (percentage), osteoblast number per bone perimeter (per mm), and osteoclast number per bone perimeter (per mm) was carried out according to standardized protocols using the Osteomeasure histomorphometry system (Osteometrix, Atlanta, GA). For assessment of dynamic histomorphometric indexes, mice were injected with calcein according to a standard double labeling protocol within 7 days. Fluorochrome measurements were made on two nonconsecutive 12-µm thick unstained sections per animal. Statistical analysis was performed using Student's t test, P < 0.05 was accepted as significant; error bars represent the SD.

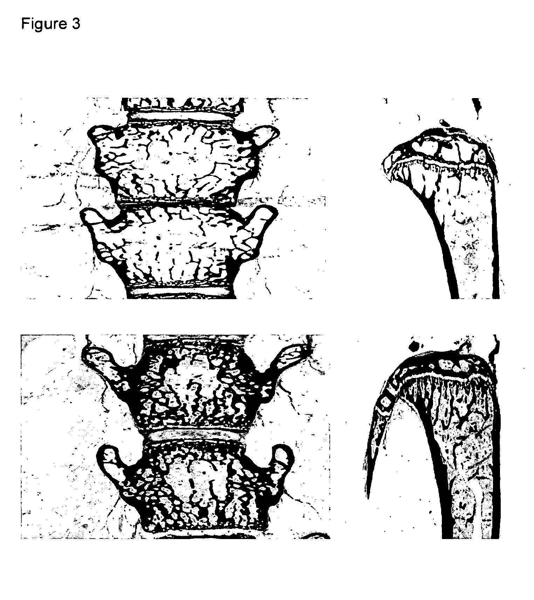

Figure 3 depicts von Kossa staining of vertebral bodies and tibia in adult wild-type and Dbc1 deficient mice. The comparison reveals that the mutant mice exhibit a calcified extra-cellular matrix which is increased by 251% relative to wild-type mice.

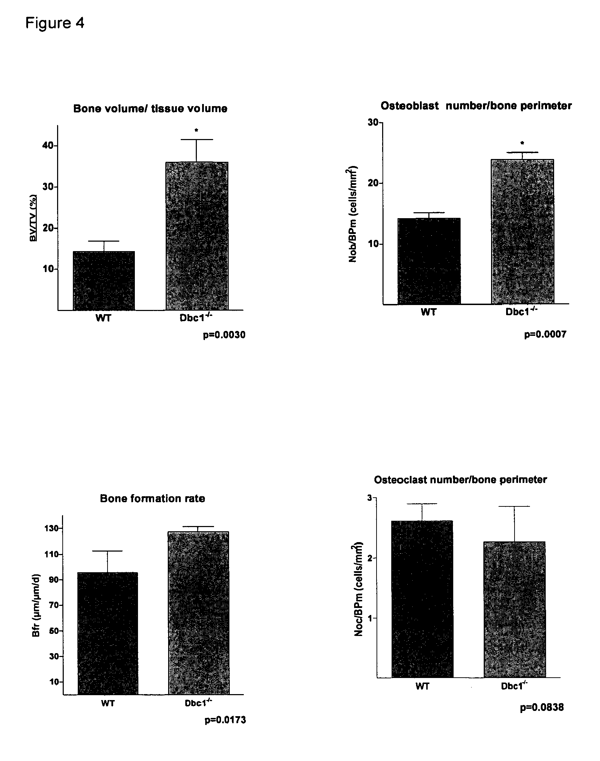

Figure 4 shows that Dbc1 regulates the number of osteoblasts. The comparison of vertebral bodies in adult wild-type and Dbc1 deficient mice shows an increase in bone substance as a ratio of bone substance to tissue substance (BV/TV). This change is accompanied by an increase in the number of osteoblasts (Nob/Bpm) and of the bone formation rate (Bfr) in Dbc1 deficient mice. The number of osteoclasts (Noc/Bpm), however, is not changed in mutant mice.

### References

1. Asubel F.M., Brent R., Kingston R.E., Moore, D.D., Seidman, J.G., Smith, Struhl, K. (1994-2008). Current protocols in molecular biology. John Wiley & Sons, Inc., Hoboken, NJ, USA.
2. Beausoleil S.A., Jedrychowski M., Schwartz D., Elias J.E., Villén J., Li J., Cohn M.A., Cantley L.C., Gygi S.P. (2004). Large-scale characterization of HeLa cell nuclear phosphoproteins. Proc Natl Acad Sci U S A. 101:12130-12135.
3. Bonifacino J.S., Dasso M., Harford, J.B., Lippincott-Schwartz, J., Yamada K. (1998-2008). Current protocols in cell biology. John Wiley & Sons, Inc., Hoboken, NJ, USA.
4. Ballif B.A., Villén J., Beausoleil S.A., Schwartz D., Gygi S.P. (2004). Phosphoproteomic analysis of the developing mouse brain. Mol Cell Proteomics. 3:1093-1101.
5. Günther T., Poli C., Müller J.M., Catala-Lehnen P., Schinke T., Yin N., Vomstein S., Amling M., Schüle R. (2005). Fhl2 deficiency results in osteopenia due to decreased activity of osteoblasts. EMBO J. 24:3049-3056.
6. Hamaguchi, M., Meth, J. L., von Klitzing, C., Wei, W., Esposito, D., Rodgers, L., Walsh, T., Welcsh, P., King, M.-C., Wigler, M. H. (2002). DBC2, a candidate for a tumor suppressor gene involved in breast cancer. Proc. Nat. Acad. Sci. 99:13647-13652.
7. Izumi, H., Inoue, J., Yokoi, S., Hosoda, H., Shibata, T., Sunamori, M., Hirohashi, S., Inazawa, J., Imoto, I. (2005). Frequent silencing of DBC1 is by genetic or epigenetic mechanisms in non-small cell lung cancers. Hum. Molec. Genet. 14: 997-1007.
8. Karsenty G., Wagner E.F. (2002). Reaching a genetic and molecular understanding of skeletal development. Dev Cell. 2:389-406.
9. Kim, J.-E., Chen, J., und Lou, Z. (2008). DBC1 is a negative regulator of SIRT1. Nature 451:583-586.
10. Molina H., Horn D.M., Tang N., Mathivanan S., Pandey A. (2007). Global proteomic profiling of phosphopeptides using electron transfer dissociation tandem mass spectrometry. Proc. Natl. Acad. Sci. U S A. 104:2199-2204.
11. Müller J. M., Isele U., Metzger E., Rempel A., Moser M., Pscherer A., Breyer T., Holubarsch C., Buettner R., Schüle R. (2000). FHL2, a novel tissue-specific coactivator of the androgen receptor. EMBO J. 19:359-369.
12. Nagase, T., Kikuno, R., Ohara, O. (2001). Prediction of the coding sequences of unidentified human genes. XXII. The complete sequences of 50 new cDNA clones which code for large proteins. DNA Res. 8:319-327.
13. Nord, A.S., Chang, P.J., Conklin, B.R., Cox, A.V., Harper, C.A., Hicks, G.G., Huang, C.C., Johns, S.J., Kawamoto, M., Liu., S., Meng, E.C., Morris, J.H., Rossant, J., Ruiz, P., Skarnes, W.C., Soriano, P., Stanford, W.L., Stryke, D., von Melchner H., Wurst, W., Yamamura, K., Young, S.G., Babbitt, P.C., Ferrin, T.E. (2006). The International Gene Trap Consortium Website: a portal to all publicly available gene trap cell lines in mouse. Nucleic Acids Res. 34:D642-648.
14. Teitelbaum S. L. (2000). Bone resorption by osteoclasts. Science. 289:1504-1508.
15. Trauernicht A.M., Kim S.J., Kim N.H., Boyer T.G. (2007). Modulation of estrogen receptor alpha protein level and survival function by DBC-1. Mol. Endocrinol. 21:1526-1536.
16. Zhao, W., Kruse, J.-P., Tang, Y., Jung, S. Y., Qin, J., Gu, W. (2008). Negative regulation of the deacetylase SIRT1 by DBC1. Nature 451:587-590.

## Claims

1. The use of a DBC1 inhibitor for the manufacture of a medicament for the treatment and/or prevention of a bone disorder.

2. The use of claim 1, wherein said DBC1 inhibitor is capable of inhibiting expression of the DBC1 gene in a cell.

3. The use of claim 2, wherein said DBC1 inhibitor is selected from siRNA, shRNA, miRNA and antisense nucleic acids.

4. The use of claim 1, wherein said DBC1 inhibitor is capable of inhibiting the binding of DBC1 protein to the osteocalcin promoter.

5. The use of claim 4, wherein said DBC1 inhibitor is an antibody specifically binding to the DBC1 protein.

6. The use of a DBC1 nucleic acid for the manufacture of a medicament for the treatment and/or prevention of a bone disorder wherein the DBC1 nucleic acid is selected from the group consisting of (a) polynucleotides comprising the sequence as shown in SEQ ID NO:1 or 3; (b) polynucleotides comprising a sequence which has an identity of at least 50% to the sequence as shown in SEQ ID NO:1; (c) polynucleotides hybridizing to the sequence as shown in SEQ ID NO:1 under stringent conditions; (d) polynucleotides comprising a sequence which encodes a polypeptide having an amino acid sequence as shown in SEQ ID NO:2; and (e) polynucleotides comprising a sequence which encodes a polypeptide having an amino acid sequence which has an identity of at least 70% to the amino acid sequence as shown in SEQ ID NO:2.

7. The use according to claim 6 wherein the DBC1 nucleic acid is a polynucleotide encoding a polypeptide having an amino acid sequence as shown in SEQ ID NO:2.

8. The use according to claim 6 or 7 wherein the DBC1 nucleic acid is a polynucleotide comprising the sequence as shown in SEQ ID NO:1.

9. The use according to any one of claims 1 to 8 wherein the bone disorder is **characterized by** a decreased bone mass relative to that of non-diseased bone.

10. The use according to any one of claims 1 to 9 wherein the bone disorder is osteoporosis.

11. A method for identifying putative therapeutic agents that may be useful for the treatment of osteoporosis, comprising:
(a) providing a candidate compound;
(b) assaying for the ability of the candidate compound to inhibit (i) expression of the Dbc1 gene in a cell, or (ii) the binding of the DBC1 protein to the osteocalcin promoter.

12. A method for the preparation of a compound that is useful in the treatment of a bone disorder, said method comprising : (a) identifying a compound by the method according to claim 11; and (b) synthesizing the compound.

13. A method of diagnosing a bone disorder, comprising (a) determining in vitro the level of expression of the Dbc1 gene in tissue from an individual; and (b) comparing the level determined in (a) to the level of expression of the Dbc1 gene in control tissue; so that if the level determined in (a) is significantly different from that of the control, the individual is diagnosed as exhibiting the bone disorder.

14. A method according to claim 15 wherein the bone disorder is osteoporosis.

15. A transgenic non-human animal **characterized by** a decreased level of expression of the Dbc1 gene relative to that of the corresponding wild-type animal.

16. The transgenic animal of claim 15 whose germ cells comprise a homozygous null mutation in the endogenous nucleic acid sequence encoding DBC1.

17. The use of the transgenic non-human animal according to claim 15 or 16 as an osteosclerosis model.

18. A method for identifying a putative agent for reducing bone mass, said method comprising: (a) administering a candidate compound to a transgenic non-human animal according to claim 15 or 16; (b) comparing the bone formation rate or the bone density of said transgenic animal to the bone formation rate or the bone density of a control animal that has not been contacted with the candidate compound; and (c) selecting the candidate compound if the bone formation rate or the bone density in the transgenic animal is significantly lower than that in the control animal.


SEQUENCE LISTING

<110>  Universitaetsklinikum Freiburg

<120>  Methods of treating bone disorders

<130>  Dbc1

<160>  12

<170>  PatentIn version 3.5

<210>  1
<211>  4031
<212>  DNA
<213>  homo sapiens


<220>
<221>  CDS
<222>  (420)..(3191)

<400>  1
cctcagtcct cccttggtgg cctcgccgcg gctctgctgg gagacccggc gctggcggct      60

gctgaggccg gagcggaggg cccgggagcg tgggagcagg agggtggcgc gcagccggtt     120

tcgcgtttgg tcggccaggg agctgccttc ccaccagccg gtcgaggaaa caacgggtcg     180

ggcttccggg gagagggccc acacagtctc ctcgccggca ccggcctcct ccatttttcc     240

gggccttgcg tggagggttt tggcggatgt ttttgaacga aggaatgtca tcggggctct     300

ctcgagcccc tcaccccgcc agctactctg ggggtggcgt ggcatagtga gaggagcgct     360

aagttgttgg gaggcctggg tccttttccc cacgactctg aaagaggaca gcgttccca     419

atg tcc cag ttt aag cgc cag cgg atc aac ccg ctt cca ggg gga cgc       467
Met Ser Gln Phe Lys Arg Gln Arg Ile Asn Pro Leu Pro Gly Gly Arg
1               5                   10                  15

aac ttc tca ggc aca gct tca aca tct ctt ctg ggc cct cct cct ggt       515
Asn Phe Ser Gly Thr Ala Ser Thr Ser Leu Leu Gly Pro Pro Pro Gly
            20                  25                  30

ttg ctc act cct cct gtg gcc aca gaa ctg tcc cag aat gcc agg cac       563
Leu Leu Thr Pro Pro Val Ala Thr Glu Leu Ser Gln Asn Ala Arg His
        35                  40                  45

ctt cag ggt ggg gag aaa cag cgg gtc ttc act ggt att gtt acc agc       611
Leu Gln Gly Gly Glu Lys Gln Arg Val Phe Thr Gly Ile Val Thr Ser
    50                  55                  60

ttg cat gac tac ttt ggg gtt gtg gat gaa gag gtc ttt ttt cag cta       659
Leu His Asp Tyr Phe Gly Val Val Asp Glu Glu Val Phe Phe Gln Leu
65                  70                  75                  80

agt gtg gtg aag ggc cgt ctg ccc cag ctg ggt gag aag gtg ctg gtg       707
Ser Val Val Lys Gly Arg Leu Pro Gln Leu Gly Glu Lys Val Leu Val
                85                  90                  95

aag gct gca tac aac cca ggc cag gca gtg ccc tgg aat gct gtc aag       755
Lys Ala Ala Tyr Asn Pro Gly Gln Ala Val Pro Trp Asn Ala Val Lys
            100                 105                 110

gtg caa acg ctc tcc aac cag ccc cta ctg aag tcc cca gca cct cct       803
Val Gln Thr Leu Ser Asn Gln Pro Leu Leu Lys Ser Pro Ala Pro Pro
        115                 120                 125

ctt ctg cat gta gca gcc ctg ggc cag aag caa ggg atc ctg gga gct 851
Leu Leu His Val Ala Ala Leu Gly Gln Lys Gln Gly Ile Leu Gly Ala
    130                 135                 140

cag cct cag ttg atc ttc cag cct cac cgg att ccc cca ctc ttt cct 899
Gln Pro Gln Leu Ile Phe Gln Pro His Arg Ile Pro Pro Leu Phe Pro
145                 150                 155                 160

cag aag cct ctg agt ctc ttc caa aca tcc cac aca ctt cac ctg agc 947
Gln Lys Pro Leu Ser Leu Phe Gln Thr Ser His Thr Leu His Leu Ser
                165                 170                 175

cac ctg aac aga ttt cct gcc cgg ggc cct cat gga cgg ttg gat cag 995
His Leu Asn Arg Phe Pro Ala Arg Gly Pro His Gly Arg Leu Asp Gln
            180                 185                 190

ggc cga agt gat gac tat gac tcc aag aaa cgc aaa cag cgg gct ggt 1043
Gly Arg Ser Asp Asp Tyr Asp Ser Lys Lys Arg Lys Gln Arg Ala Gly
        195                 200                 205

gga gag ccc tgg ggt gct aag aag cca agg cat gac ctg cct cct tac 1091
Gly Glu Pro Trp Gly Ala Lys Lys Pro Arg His Asp Leu Pro Pro Tyr
    210                 215                 220

cgg gtc cac ctc act cct tac act gtg gac agc ccc atc tgt gac ttc 1139
Arg Val His Leu Thr Pro Tyr Thr Val Asp Ser Pro Ile Cys Asp Phe
225                 230                 235                 240

cta gaa ctc cag cgc cgt tac cgc agc ctc ctg gtc ccc tca gat ttt 1187
Leu Glu Leu Gln Arg Arg Tyr Arg Ser Leu Leu Val Pro Ser Asp Phe
                245                 250                 255

ctg tcc gtg cat ctg agt tgg cta tca gcc ttc ccc ctg agc cag ccc 1235
Leu Ser Val His Leu Ser Trp Leu Ser Ala Phe Pro Leu Ser Gln Pro
            260                 265                 270

ttt tcc ctc cat cat cca agc cgg atc cag gtc tct tct gaa aag gag 1283
Phe Ser Leu His His Pro Ser Arg Ile Gln Val Ser Ser Glu Lys Glu
        275                 280                 285

gca gct cca gac gct ggt gct gag ccc atc act gca gac agt gac ccc 1331
Ala Ala Pro Asp Ala Gly Ala Glu Pro Ile Thr Ala Asp Ser Asp Pro
    290                 295                 300

gct tat agt tcg aag gta ctg ctg ctc tct tcc ccg ggg ttg gag gaa 1379
Ala Tyr Ser Ser Lys Val Leu Leu Leu Ser Ser Pro Gly Leu Glu Glu
305                 310                 315                 320

ttg tat cgt tgt tgc atg ctc ttt gtg gat gac atg gct gag cca agg 1427
Leu Tyr Arg Cys Cys Met Leu Phe Val Asp Asp Met Ala Glu Pro Arg
                325                 330                 335

gag acg cca gag cat cct ctg aag cag att aag ttt ttg ctg ggc agg 1475
Glu Thr Pro Glu His Pro Leu Lys Gln Ile Lys Phe Leu Leu Gly Arg
            340                 345                 350

aaa gaa gag gag gca gtg ctg gtt ggg ggt gaa tgg tct cct tcc ctg 1523
Lys Glu Glu Glu Ala Val Leu Val Gly Gly Glu Trp Ser Pro Ser Leu
        355                 360                 365

gat ggc ctc gac ccc cag gct gac ccg cag gtg ctg gtg cgt acc gcc 1571
Asp Gly Leu Asp Pro Gln Ala Asp Pro Gln Val Leu Val Arg Thr Ala
    370                 375                 380

atc cgc tgt gcg cag gcc cag act ggc att gat ttg agc ggc tgt acc 1619
Ile Arg Cys Ala Gln Ala Gln Thr Gly Ile Asp Leu Ser Gly Cys Thr
385                 390                 395                 400

aag tgg tgg cgc ttt gcc gag ttt cag tac ctg cag ccg gga ccc ccc 1667

Lys Trp Trp Arg Phe Ala Glu Phe Gln Tyr Leu Gln Pro Gly Pro Pro
                405                 410                 415

cgg cgg ctt cag aca gtg gtg gtg tac ctg ccg gat gtc tgg acc atc      1715
Arg Arg Leu Gln Thr Val Val Val Tyr Leu Pro Asp Val Trp Thr Ile
            420                 425                 430

atg cct act ttg gag gag tgg gag gcc ctg tgc cag cag aaa gct gca      1763
Met Pro Thr Leu Glu Glu Trp Glu Ala Leu Cys Gln Gln Lys Ala Ala
        435                 440                 445

gag gca gct ccc cca acc cag gag gca caa ggg gaa acg gag cct act      1811
Glu Ala Ala Pro Pro Thr Gln Glu Ala Gln Gly Glu Thr Glu Pro Thr
    450                 455                 460

gaa cag gca cct gat gcc ttg gag caa gca gca gac act tct aga cgg      1859
Glu Gln Ala Pro Asp Ala Leu Glu Gln Ala Ala Asp Thr Ser Arg Arg
465                 470                 475                 480

aac gca gaa act cca gag gcc acc aca cag cag gaa acg gac act gat      1907
Asn Ala Glu Thr Pro Glu Ala Thr Thr Gln Gln Glu Thr Asp Thr Asp
                485                 490                 495

ctc cca gag gcc cct cca ccc ccc cta gaa cct gct gtc atc gca cgc      1955
Leu Pro Glu Ala Pro Pro Pro Pro Leu Glu Pro Ala Val Ile Ala Arg
            500                 505                 510

cct ggc tgt gta aac ctg tcc ctc cat ggg att gtg gag gat cgg agg      2003
Pro Gly Cys Val Asn Leu Ser Leu His Gly Ile Val Glu Asp Arg Arg
        515                 520                 525

cca aag gaa agg atc tct ttt gag gtg atg gtg ctg gcc gag ctg ttt      2051
Pro Lys Glu Arg Ile Ser Phe Glu Val Met Val Leu Ala Glu Leu Phe
    530                 535                 540

ctg gag atg ctc cag agg gat ttt ggc tat aga gtt tat aag atg cta      2099
Leu Glu Met Leu Gln Arg Asp Phe Gly Tyr Arg Val Tyr Lys Met Leu
545                 550                 555                 560

ctg agc ctt cct gaa aag gtc gtg tcc cca cct gaa cct gag aag gag      2147
Leu Ser Leu Pro Glu Lys Val Val Ser Pro Pro Glu Pro Glu Lys Glu
                565                 570                 575

gag gcg gcc aag gaa gaa gcc acc aag gag gaa gaa gcc atc aaa gag      2195
Glu Ala Ala Lys Glu Glu Ala Thr Lys Glu Glu Glu Ala Ile Lys Glu
            580                 585                 590

gag gtg gtc aag gag ccc aag gat gag gca cag aat gag ggc ccg gct      2243
Glu Val Val Lys Glu Pro Lys Asp Glu Ala Gln Asn Glu Gly Pro Ala
        595                 600                 605

aca gag tca gag gcc ccg ctg aag gag gat ggg ctt ttg ccc aaa cca      2291
Thr Glu Ser Glu Ala Pro Leu Lys Glu Asp Gly Leu Leu Pro Lys Pro
    610                 615                 620

ctc tct tct ggg gga gag gaa gaa gaa aaa ccc cgg ggc gag gct tct      2339
Leu Ser Ser Gly Gly Glu Glu Glu Glu Lys Pro Arg Gly Glu Ala Ser
625                 630                 635                 640

gag gac ctg tgt gag atg gcc ctg gac cca gaa ctg ttg ctt ctg agg      2387
Glu Asp Leu Cys Glu Met Ala Leu Asp Pro Glu Leu Leu Leu Leu Arg
                645                 650                 655

gat gat gga gag gag gag ttt gca gga gca aag ctg gag gat tcg gag      2435
Asp Asp Gly Glu Glu Glu Phe Ala Gly Ala Lys Leu Glu Asp Ser Glu
            660                 665                 670

gtc cgg tcc gtt gcc tca aac cag tca gag atg gag ttc tct tca ctt      2483
Val Arg Ser Val Ala Ser Asn Gln Ser Glu Met Glu Phe Ser Ser Leu

        675                 680                 685

cag gac atg ccc aag gag ctg gat ccc tct gct gtg ctc ccc tta gac 2531
Gln Asp Met Pro Lys Glu Leu Asp Pro Ser Ala Val Leu Pro Leu Asp
    690                 695                 700

tgt ctg ctt gct ttt gtg ttc ttt gat gcc aac tgg tgt ggc tac ttg 2579
Cys Leu Leu Ala Phe Val Phe Phe Asp Ala Asn Trp Cys Gly Tyr Leu
705                 710                 715                 720

cac cgg cga gac tta gag agg atc ctc ctt acc ctt ggg atc cgg ctc 2627
His Arg Arg Asp Leu Glu Arg Ile Leu Leu Thr Leu Gly Ile Arg Leu
                725                 730                 735

agt gca gag cag gcc aag cag ctg gtc agc agg gtg gtg acc cag aac 2675
Ser Ala Glu Gln Ala Lys Gln Leu Val Ser Arg Val Val Thr Gln Asn
            740                 745                 750

atc tgc cag tac cgg agc ctt cag tac agc cgc cag gag ggc ctg gat 2723
Ile Cys Gln Tyr Arg Ser Leu Gln Tyr Ser Arg Gln Glu Gly Leu Asp
        755                 760                 765

ggt ggc ctt ccc gag gag gtg ctc ttc gga aac ctg gac ctg ctg ccc 2771
Gly Gly Leu Pro Glu Glu Val Leu Phe Gly Asn Leu Asp Leu Leu Pro
    770                 775                 780

cct cct ggg aaa agc acg aag cca ggt gct gcc ccc aca gaa cac aaa 2819
Pro Pro Gly Lys Ser Thr Lys Pro Gly Ala Ala Pro Thr Glu His Lys
785                 790                 795                 800

gcc ttg gtg tcc cac aat ggc agc ctg att aac gtg ggg agc ctg ctg 2867
Ala Leu Val Ser His Asn Gly Ser Leu Ile Asn Val Gly Ser Leu Leu
                805                 810                 815

cag cgc gcg gag cag cag gac agc ggc cgg ctc tac cta gag aac aag 2915
Gln Arg Ala Glu Gln Gln Asp Ser Gly Arg Leu Tyr Leu Glu Asn Lys
            820                 825                 830

atc cac aca ctg gag ctg aag ctg gag gag agc cat aac cgt ttc tca 2963
Ile His Thr Leu Glu Leu Lys Leu Glu Glu Ser His Asn Arg Phe Ser
        835                 840                 845

gcc act gaa gta acc aat aag acg ctg gcg gca gag atg cag gag ctg 3011
Ala Thr Glu Val Thr Asn Lys Thr Leu Ala Ala Glu Met Gln Glu Leu
    850                 855                 860

cga gtc cgg ctg gcg gag gcc gag gag acc gcc cgg acg gcg gag cga 3059
Arg Val Arg Leu Ala Glu Ala Glu Glu Thr Ala Arg Thr Ala Glu Arg
865                 870                 875                 880

cag aag agc cag ctc cag cgg ctg ctg cag gag ctc cgc agg cgt ctg 3107
Gln Lys Ser Gln Leu Gln Arg Leu Leu Gln Glu Leu Arg Arg Arg Leu
                885                 890                 895

acc ccc ctg cag ctg gag atc cag cgg gtg gtg gaa aag gct gac agc 3155
Thr Pro Leu Gln Leu Glu Ile Gln Arg Val Val Glu Lys Ala Asp Ser
            900                 905                 910

tgg gtg gag aag gag gag ccg gca cct agc aac tga cggcctcgca 3201
Trp Val Glu Lys Glu Glu Pro Ala Pro Ser Asn
        915                 920

cggaactgcc atcctgtgag ggcagcggtg gcgcccggca aagttggagc ccttgcggta 3261

ccagaaagca gcgagagcga gacctgggag ccagggcagg ggtggctgac cccatgctca 3321

gcctctaggg gacggcaggc catcaggctg ggggctgtgc tatgtgggat ggatgtgtga 3381

ggaaccccgg ttccacttaa caactaaata caacatcttt tgcaccccta gaatgtcatt 3441

ttgccctcaa ccttggtatt tctcctgggg cccttttagt cttgtgctga ctttctcctg 3501

tcctcttcca gtttagaata agacagggga gaaaaaggct tttcgagtgt gggacaaggt 3561

ctgatgtcag tgaacggaac tgaagagcaa gacatggagc ctggctgggg ctaagcagcc 3621

ccctcctccg gatggcacag gctctgctag gttccggaca caggcttctg ggggaagggt 3681

ctcccttggc catcacggga atggagtcca tgctagaaag agctcagtgt tgggctgggt 3741

ttgccagtag aacactgcgt tccagtcacc ccggtcttgc cagaagaaac cagcacctct 3801

ttcccgtggc tcctgtgttc agaatgtggt attggctctg gcccagcctt ctcccctgtt 3861

acccataatt cttgcctctt tccataatcc gtggtttcag tttgactttg tatataaagt 3921

tggggttttt tttttttttt tttggcttgt tttttaaata aaccaaagtc aaaaacaaaa 3981

aaaaaaaaaa aaaaaaaaaa aaaaaaaaaa aaaaaaaaaa aaaaaaaaaa 4031

<210> 2
<211> 923
<212> PRT
<213> homo sapiens

<400> 2

Met Ser Gln Phe Lys Arg Gln Arg Ile Asn Pro Leu Pro Gly Gly Arg
1               5                   10                  15

Asn Phe Ser Gly Thr Ala Ser Thr Ser Leu Leu Gly Pro Pro Pro Gly
            20                  25                  30

Leu Leu Thr Pro Pro Val Ala Thr Glu Leu Ser Gln Asn Ala Arg His
        35                  40                  45

Leu Gln Gly Gly Glu Lys Gln Arg Val Phe Thr Gly Ile Val Thr Ser
    50                  55                  60

Leu His Asp Tyr Phe Gly Val Val Asp Glu Glu Val Phe Phe Gln Leu
65                  70                  75                  80

Ser Val Val Lys Gly Arg Leu Pro Gln Leu Gly Glu Lys Val Leu Val
                85                  90                  95

Lys Ala Ala Tyr Asn Pro Gly Gln Ala Val Pro Trp Asn Ala Val Lys
            100                 105                 110

Val Gln Thr Leu Ser Asn Gln Pro Leu Leu Lys Ser Pro Ala Pro Pro
        115                 120                 125

Leu Leu His Val Ala Ala Leu Gly Gln Lys Gln Gly Ile Leu Gly Ala
    130                 135                 140

Gln Pro Gln Leu Ile Phe Gln Pro His Arg Ile Pro Pro Leu Phe Pro
145                 150                 155                 160

Gln Lys Pro Leu Ser Leu Phe Gln Thr Ser His Thr Leu His Leu Ser
                165                 170                 175

His Leu Asn Arg Phe Pro Ala Arg Gly Pro His Gly Arg Leu Asp Gln
            180                 185                 190

Gly Arg Ser Asp Asp Tyr Asp Ser Lys Lys Arg Lys Gln Arg Ala Gly
        195                 200                 205

Gly Glu Pro Trp Gly Ala Lys Lys Pro Arg His Asp Leu Pro Pro Tyr
    210                 215                 220

Arg Val His Leu Thr Pro Tyr Thr Val Asp Ser Pro Ile Cys Asp Phe
225                 230                 235                 240

Leu Glu Leu Gln Arg Arg Tyr Arg Ser Leu Leu Val Pro Ser Asp Phe
                245                 250                 255

Leu Ser Val His Leu Ser Trp Leu Ser Ala Phe Pro Leu Ser Gln Pro
            260                 265                 270

Phe Ser Leu His His Pro Ser Arg Ile Gln Val Ser Ser Glu Lys Glu
        275                 280                 285

Ala Ala Pro Asp Ala Gly Ala Glu Pro Ile Thr Ala Asp Ser Asp Pro
    290                 295                 300

Ala Tyr Ser Ser Lys Val Leu Leu Leu Ser Ser Pro Gly Leu Glu Glu
305                 310                 315                 320

Leu Tyr Arg Cys Cys Met Leu Phe Val Asp Asp Met Ala Glu Pro Arg
                325                 330                 335

Glu Thr Pro Glu His Pro Leu Lys Gln Ile Lys Phe Leu Leu Gly Arg
            340                 345                 350

Lys Glu Glu Glu Ala Val Leu Val Gly Gly Glu Trp Ser Pro Ser Leu
        355                 360                 365

Asp Gly Leu Asp Pro Gln Ala Asp Pro Gln Val Leu Val Arg Thr Ala
    370                 375                 380

Ile Arg Cys Ala Gln Ala Gln Thr Gly Ile Asp Leu Ser Gly Cys Thr
385                 390                 395                 400

Lys Trp Trp Arg Phe Ala Glu Phe Gln Tyr Leu Gln Pro Gly Pro Pro
                405                 410                 415

Arg Arg Leu Gln Thr Val Val Val Tyr Leu Pro Asp Val Trp Thr Ile
            420                 425                 430

Met Pro Thr Leu Glu Glu Trp Glu Ala Leu Cys Gln Gln Lys Ala Ala

435                 440                 445

Glu Ala Ala Pro Pro Thr Gln Glu Ala Gln Gly Glu Thr Glu Pro Thr
    450                 455                 460

Glu Gln Ala Pro Asp Ala Leu Glu Gln Ala Ala Asp Thr Ser Arg Arg
465                 470                 475                 480

Asn Ala Glu Thr Pro Glu Ala Thr Thr Gln Gln Glu Thr Asp Thr Asp
                485                 490                 495

Leu Pro Glu Ala Pro Pro Pro Pro Leu Glu Pro Ala Val Ile Ala Arg
            500                 505                 510

Pro Gly Cys Val Asn Leu Ser Leu His Gly Ile Val Glu Asp Arg Arg
        515                 520                 525

Pro Lys Glu Arg Ile Ser Phe Glu Val Met Val Leu Ala Glu Leu Phe
    530                 535                 540

Leu Glu Met Leu Gln Arg Asp Phe Gly Tyr Arg Val Tyr Lys Met Leu
545                 550                 555                 560

Leu Ser Leu Pro Glu Lys Val Val Ser Pro Pro Glu Pro Glu Lys Glu
                565                 570                 575

Glu Ala Ala Lys Glu Glu Ala Thr Lys Glu Glu Glu Ala Ile Lys Glu
            580                 585                 590

Glu Val Val Lys Glu Pro Lys Asp Glu Ala Gln Asn Glu Gly Pro Ala
        595                 600                 605

Thr Glu Ser Glu Ala Pro Leu Lys Glu Asp Gly Leu Leu Pro Lys Pro
    610                 615                 620

Leu Ser Ser Gly Gly Glu Glu Glu Lys Pro Arg Gly Glu Ala Ser
625                 630                 635                 640

Glu Asp Leu Cys Glu Met Ala Leu Asp Pro Glu Leu Leu Leu Leu Arg
                645                 650                 655

Asp Asp Gly Glu Glu Glu Phe Ala Gly Ala Lys Leu Glu Asp Ser Glu
            660                 665                 670

Val Arg Ser Val Ala Ser Asn Gln Ser Glu Met Glu Phe Ser Ser Leu
        675                 680                 685

Gln Asp Met Pro Lys Glu Leu Asp Pro Ser Ala Val Leu Pro Leu Asp
    690                 695                 700

Cys Leu Leu Ala Phe Val Phe Phe Asp Ala Asn Trp Cys Gly Tyr Leu
705                 710                 715                 720

His Arg Arg Asp Leu Glu Arg Ile Leu Leu Thr Leu Gly Ile Arg Leu
                725                 730                 735

Ser Ala Glu Gln Ala Lys Gln Leu Val Ser Arg Val Val Thr Gln Asn
            740                 745                 750

Ile Cys Gln Tyr Arg Ser Leu Gln Tyr Ser Arg Gln Glu Gly Leu Asp
        755                 760                 765

Gly Gly Leu Pro Glu Glu Val Leu Phe Gly Asn Leu Asp Leu Leu Pro
    770                 775                 780

Pro Pro Gly Lys Ser Thr Lys Pro Gly Ala Ala Pro Thr Glu His Lys
785                 790                 795                 800

Ala Leu Val Ser His Asn Gly Ser Leu Ile Asn Val Gly Ser Leu Leu
                805                 810                 815

Gln Arg Ala Glu Gln Gln Asp Ser Gly Arg Leu Tyr Leu Glu Asn Lys
            820                 825                 830

Ile His Thr Leu Glu Leu Lys Leu Glu Glu Ser His Asn Arg Phe Ser
        835                 840                 845

Ala Thr Glu Val Thr Asn Lys Thr Leu Ala Ala Glu Met Gln Glu Leu
    850                 855                 860

Arg Val Arg Leu Ala Glu Ala Glu Glu Thr Ala Arg Thr Ala Glu Arg
865                 870                 875                 880

Gln Lys Ser Gln Leu Gln Arg Leu Leu Gln Glu Leu Arg Arg Arg Leu
                885                 890                 895

Thr Pro Leu Gln Leu Glu Ile Gln Arg Val Val Glu Lys Ala Asp Ser
            900                 905                 910

Trp Val Glu Lys Glu Glu Pro Ala Pro Ser Asn
        915                 920

<210>  3
<211>  3772
<212>  DNA
<213>  mus muscululs


<220>
<221>  CDS
<222>  (232)..(3000)

<400>  3
ccacgcgtcc gatggcaggc tggtgtgggg gaagggagag gtcgatggag aagagggagg      60

taccatatcc gggagagtgg cagcttccga ccagtggtct ctcttccgga gaggcgtttc     120

cggtggtggt ggcagcggcg gctgcggtgg ttccgggtgt ctttgtcccc ctggtgtcgt 180

ggccctggcc cgcaggtttc cccccaagac cttgaaagga cagtattcca a atg tcc 237
Met Ser
1

cag ttt aag cgg cag agg atc aac cca ctt cca ggg gga cgc aac ttc 285
Gln Phe Lys Arg Gln Arg Ile Asn Pro Leu Pro Gly Gly Arg Asn Phe
        5                   10                  15

tca ggc gca gct tca aca tct ctt ttg ggc cct ccc cct ggt cta ctt 333
Ser Gly Ala Ala Ser Thr Ser Leu Leu Gly Pro Pro Pro Gly Leu Leu
    20                  25                  30

act cct cct gtg gcc aca gac ctg tcc caa aat gcc agg cac ctt cag 381
Thr Pro Pro Val Ala Thr Asp Leu Ser Gln Asn Ala Arg His Leu Gln
35                  40                  45                  50

agt ggg gaa aag cag cga gtc ttc aca ggc att gtt acc agc ttg cat 429
Ser Gly Glu Lys Gln Arg Val Phe Thr Gly Ile Val Thr Ser Leu His
                55                  60                  65

gac tac ttt ggg gtg gta gat gaa gaa gtc ttt ttt cag cta agt gtg 477
Asp Tyr Phe Gly Val Val Asp Glu Glu Val Phe Phe Gln Leu Ser Val
            70                  75                  80

gtg aag ggt cga ctg ccc cag ctg ggg gag aag gtg ctg gtg aag gcc 525
Val Lys Gly Arg Leu Pro Gln Leu Gly Glu Lys Val Leu Val Lys Ala
        85                  90                  95

gca tac aac cca ggc cag gcc gtg ccc tgg aat gct gtc aag gtg cag 573
Ala Tyr Asn Pro Gly Gln Ala Val Pro Trp Asn Ala Val Lys Val Gln
    100                 105                 110

acg ctc tcc aac cag ccc tta ctg aag tct cca gcg cct ccc ctt ctg 621
Thr Leu Ser Asn Gln Pro Leu Leu Lys Ser Pro Ala Pro Pro Leu Leu
115                 120                 125                 130

cac gtg gca gct ctg ggc cag aag caa ggg atc ctg gga gct cag ccc 669
His Val Ala Ala Leu Gly Gln Lys Gln Gly Ile Leu Gly Ala Gln Pro
                135                 140                 145

cag ctg atc ttt cag cct cac cga att ccc ccc ctt ttc cct cag aag 717
Gln Leu Ile Phe Gln Pro His Arg Ile Pro Pro Leu Phe Pro Gln Lys
            150                 155                 160

cct ctg agt ctc ttc cag aca tcc cac aca ctt cac ctg agc cac ctg 765
Pro Leu Ser Leu Phe Gln Thr Ser His Thr Leu His Leu Ser His Leu
        165                 170                 175

aac aga ttt cct gct cgg ggt cct cat gga agg ctg gac cag ggc cga 813
Asn Arg Phe Pro Ala Arg Gly Pro His Gly Arg Leu Asp Gln Gly Arg
    180                 185                 190

agt gat gac tat gac tcg aag aaa cgc aaa cag cgg gct ggc gga gag 861
Ser Asp Asp Tyr Asp Ser Lys Lys Arg Lys Gln Arg Ala Gly Gly Glu
195                 200                 205                 210

cct tgg ggt gct aag aaa cct agg cat gac ctg tct cct tac cgg gtt 909
Pro Trp Gly Ala Lys Lys Pro Arg His Asp Leu Ser Pro Tyr Arg Val
                215                 220                 225

cat ctc act ccc tat act gtg gac agt ccc acc tgt gac ttc cta gaa 957
His Leu Thr Pro Tyr Thr Val Asp Ser Pro Thr Cys Asp Phe Leu Glu
            230                 235                 240

ctg cag cga cgt tac cgc agc ctg ttg gtc ccc tca gat ttc ctt tcc 1005
Leu Gln Arg Arg Tyr Arg Ser Leu Leu Val Pro Ser Asp Phe Leu Ser
        245                 250                 255

gtt cac ttg agc tgg ctg tca gcc ttc cct ctg ggc cag ccc ttt tcc 1053
Val His Leu Ser Trp Leu Ser Ala Phe Pro Leu Gly Gln Pro Phe Ser
    260                 265                 270

ctc cac cac cca agt cgg atc cag gtc tct tca gag aag gag gca gct 1101
Leu His His Pro Ser Arg Ile Gln Val Ser Ser Glu Lys Glu Ala Ala
275                 280                 285                 290

cca gac act ggt gct gag ccc agc cct gag gac agt gac ccc act tac 1149
Pro Asp Thr Gly Ala Glu Pro Ser Pro Glu Asp Ser Asp Pro Thr Tyr
                295                 300                 305

agt tcc aag gtg ctg ctg ctc tcc tcc cca ggc ctg gag gag ttc tat 1197
Ser Ser Lys Val Leu Leu Leu Ser Ser Pro Gly Leu Glu Glu Phe Tyr
            310                 315                 320

cgt tgc tgc atg ctg ttt gtg gat gac atg gct gag cca agg gag aca 1245
Arg Cys Cys Met Leu Phe Val Asp Asp Met Ala Glu Pro Arg Glu Thr
        325                 330                 335

cca gag cat cct ctg aag cag cta aag ttt ttg ctg ggc cgg aaa gaa 1293
Pro Glu His Pro Leu Lys Gln Leu Lys Phe Leu Leu Gly Arg Lys Glu
    340                 345                 350

gaa gag gca gtg ctg gtt ggg ggt gag tgg tct cct tcc ctg gat ggc 1341
Glu Glu Ala Val Leu Val Gly Gly Glu Trp Ser Pro Ser Leu Asp Gly
355                 360                 365                 370

ctc gac ccc cag gca gac ccg cag gtg ctg gtg cgc aca gcc atc cgc 1389
Leu Asp Pro Gln Ala Asp Pro Gln Val Leu Val Arg Thr Ala Ile Arg
                375                 380                 385

tgt gca caa gcc cag act ggc att gac ttg agc acc tgc acc aaa tgg 1437
Cys Ala Gln Ala Gln Thr Gly Ile Asp Leu Ser Thr Cys Thr Lys Trp
            390                 395                 400

tgg cgc ttt gct gag ttt cag tac tta cag ccg ggg ccg ccc cgg cag 1485
Trp Arg Phe Ala Glu Phe Gln Tyr Leu Gln Pro Gly Pro Pro Arg Gln
        405                 410                 415

tta cac aca gta gtg gtg tac ctg cca gac gtg tgg acc atc atg ccc 1533
Leu His Thr Val Val Val Tyr Leu Pro Asp Val Trp Thr Ile Met Pro
    420                 425                 430

act ttg gag gag tgg gag gca ctg tgc caa cag aaa gcc acg gag gca 1581
Thr Leu Glu Glu Trp Glu Ala Leu Cys Gln Gln Lys Ala Thr Glu Ala
435                 440                 445                 450

gct ccc caa cca cat gag gca tca ggg gaa gca gag gct act gaa cag 1629
Ala Pro Gln Pro His Glu Ala Ser Gly Glu Ala Glu Ala Thr Glu Gln
                455                 460                 465

gct cct gat gtg tca gag caa gca gac act tct aaa cag aac aca gag 1677
Ala Pro Asp Val Ser Glu Gln Ala Asp Thr Ser Lys Gln Asn Thr Glu
            470                 475                 480

aca atg gag gcc acc aca cag caa gat gtg gac act gat ctc cca gag 1725
Thr Met Glu Ala Thr Thr Gln Gln Asp Val Asp Thr Asp Leu Pro Glu
        485                 490                 495

gcc cct ccg cct cct cta gaa cct gct gtg atg gcc cgt cct cgc tgt 1773
Ala Pro Pro Pro Pro Leu Glu Pro Ala Val Met Ala Arg Pro Arg Cys
    500                 505                 510

gtc aac ctg tct ctt tat gga att gtg gag gat cgg agg cca aaa gaa 1821
Val Asn Leu Ser Leu Tyr Gly Ile Val Glu Asp Arg Arg Pro Lys Glu
515                 520                 525                 530

agg atc tct ttt gag gta gtg gtg ctg gct gag ctg ttt gta gag atg 1869
Arg Ile Ser Phe Glu Val Val Val Leu Ala Glu Leu Phe Val Glu Met
                535                 540                 545

ctg cag agg gat ttt ggc tat aga att tat aag acg ctg ctg agc ctt 1917
Leu Gln Arg Asp Phe Gly Tyr Arg Ile Tyr Lys Thr Leu Leu Ser Leu
            550                 555                 560

cca gaa aaa gtt gtg tct cct cct gaa cct gag aag gag gag gca gcc 1965
Pro Glu Lys Val Val Ser Pro Pro Glu Pro Glu Lys Glu Glu Ala Ala
        565                 570                 575

aaa gaa gat gcg gtc aaa gag gag gag gct gtc aaa gag gag gca gtg 2013
Lys Glu Asp Ala Val Lys Glu Glu Glu Ala Val Lys Glu Glu Ala Val
    580                 585                 590

aag gtg tcc aag gat gag gta cag aat gag ggc aca gct gcc gag tca 2061
Lys Val Ser Lys Asp Glu Val Gln Asn Glu Gly Thr Ala Ala Glu Ser
595                 600                 605                 610

gac agc cca ctg aag gag gat ggg ctt ctg ccc aaa cgg ccc tct tca 2109
Asp Ser Pro Leu Lys Glu Asp Gly Leu Leu Pro Lys Arg Pro Ser Ser
                615                 620                 625

ggg gga gaa gag gag gaa aag gcc cgg ggc gag gcg gct gag gac ctc 2157
Gly Gly Glu Glu Glu Glu Lys Ala Arg Gly Glu Ala Ala Glu Asp Leu
            630                 635                 640

tgt gag atg gcc ttg gac cca gac ctg ctg ctg ctg cgg gat gat gga 2205
Cys Glu Met Ala Leu Asp Pro Asp Leu Leu Leu Leu Arg Asp Asp Gly
        645                 650                 655

gag gac gaa ttt gca gga gca aag cta gag gaa aca gag gtt cgt tct 2253
Glu Asp Glu Phe Ala Gly Ala Lys Leu Glu Glu Thr Glu Val Arg Ser
    660                 665                 670

gtt gcc tca aac cag tca gag atg gaa tat tct tct ctt cag gac atg 2301
Val Ala Ser Asn Gln Ser Glu Met Glu Tyr Ser Ser Leu Gln Asp Met
675                 680                 685                 690

cct aag gag ctg gat ccc tca act gtg ctc ccc ctg gac tgt ctt ttg 2349
Pro Lys Glu Leu Asp Pro Ser Thr Val Leu Pro Leu Asp Cys Leu Leu
                695                 700                 705

gct ttt gtg ttt ttt gat gcc aac tgg tgt ggc tac ttg cac agg cga 2397
Ala Phe Val Phe Phe Asp Ala Asn Trp Cys Gly Tyr Leu His Arg Arg
            710                 715                 720

gac ttg gag agg gtc ctc ctc aca cta ggg atc cgg ctc agt gca gag 2445
Asp Leu Glu Arg Val Leu Leu Thr Leu Gly Ile Arg Leu Ser Ala Glu
        725                 730                 735

cag gcc aaa cag ttg gtt agt aga gtg gtg gca cag aac atc tgc cag 2493
Gln Ala Lys Gln Leu Val Ser Arg Val Val Ala Gln Asn Ile Cys Gln
    740                 745                 750

tac cgg agc ctt cag tac agc cgt gct gaa gtg ctg gac gat ggc ctt 2541
Tyr Arg Ser Leu Gln Tyr Ser Arg Ala Glu Val Leu Asp Asp Gly Leu
755                 760                 765                 770

cct gaa gac gtg ctc ttc gga aac ctg gat cta ctg cct cct tca ggg 2589
Pro Glu Asp Val Leu Phe Gly Asn Leu Asp Leu Leu Pro Pro Ser Gly
                775                 780                 785

aag agc aca aag cca ggt gct gcc ccc aca gag cac aaa ggc ctg gtg 2637
Lys Ser Thr Lys Pro Gly Ala Ala Pro Thr Glu His Lys Gly Leu Val
            790                 795                 800

ccc cac aat ggt agc ctc atc aat gtg gga agc ctg tta cag cgt gca 2685

Pro His Asn Gly Ser Leu Ile Asn Val Gly Ser Leu Leu Gln Arg Ala
        805                 810                 815

gag cag caa gac agt ggc cgc cta tac ctg gag aac aag att cac aca 2733
Glu Gln Gln Asp Ser Gly Arg Leu Tyr Leu Glu Asn Lys Ile His Thr
    820                 825                 830

ctg gaa ctg aag ctt gag gag agc cat aac cgc ttc tca gcc act gag 2781
Leu Glu Leu Lys Leu Glu Glu Ser His Asn Arg Phe Ser Ala Thr Glu
835                 840                 845                 850

gtg aca aat aag aca ctg gca gca gag atg cag gag ctg cgg gcc cgg 2829
Val Thr Asn Lys Thr Leu Ala Ala Glu Met Gln Glu Leu Arg Ala Arg
                855                 860                 865

ctg gcc gag gca gag gag acg gcc cgg aca gca gag cga cag aag aac 2877
Leu Ala Glu Ala Glu Glu Thr Ala Arg Thr Ala Glu Arg Gln Lys Asn
            870                 875                 880

cag ctc cag cga cag atg cag gac ttc cgc agg cgc ctg acc cca ctg 2925
Gln Leu Gln Arg Gln Met Gln Asp Phe Arg Arg Arg Leu Thr Pro Leu
        885                 890                 895

cac ctt gag atg cag cgg att gtt gag aag gct gac agc tgg gta gag 2973
His Leu Glu Met Gln Arg Ile Val Glu Lys Ala Asp Ser Trp Val Glu
    900                 905                 910

aaa gaa gag cca acg cct agc aac tga gtggctgcct agtgaggtct 3020
Lys Glu Glu Pro Thr Pro Ser Asn
915                 920

gcgatggagc ctgatgacat tagagccctt ttggtaccag aaggcagctg gagcagggcc 3080

tgggagccac agcaggggta gcttgagttc tgtgctcagc ctctgcaggg gatgtcaaac 3140

tcagtatggg gtttgtgcta tgtggatgga tatgtaagga actccagttc cagctacaac 3200

taaatacaac gtcctttgca cccctagaat gtcattttgc cctcaatctt ggaatttctc 3260

ccagggccct tgtagtcttg tgctgtctcc tgtcctcgtc cattttagag caagacggga 3320

ggaaaaggca tttccagccc agaacaaggt ctaatgccaa taaatgggag gaatgggtgt 3380

agagttgatg cagccaagaa tgtccgtcct ctgggcggca caagtcccgc aggctctgtg 3440

tctgttaggt gatggagccc attcaaggag catttggtgt tgggtcaggg ttgccaatag 3500

aacactgtgt cccagctgcc cccaacttgc cagaagaacc agcacttact tctttctcta 3560

gttcttatgt tcagaatgtg gtattggccc tggcctagcc ccttccactt cccataattc 3620

ttgcctcttt ccataattca tggtttcagt tcgactttgt atataaagtt gtttgttggt 3680

tttttttttt tttttttggc tttttgtttt ttaaataaac caaagtgaaa aacaaaaaaa 3740

aaaaaaaaaa aaaaaaaaaa aaaaaaaaaa aa 3772

<210> 4
<211> 922
<212> PRT
<213> mus muscululs

<400> 4

Met Ser Gln Phe Lys Arg Gln Arg Ile Asn Pro Leu Pro Gly Gly Arg
1               5                   10                  15

Asn Phe Ser Gly Ala Ala Ser Thr Ser Leu Leu Gly Pro Pro Pro Gly
            20                  25                  30

Leu Leu Thr Pro Pro Val Ala Thr Asp Leu Ser Gln Asn Ala Arg His
        35                  40                  45

Leu Gln Ser Gly Glu Lys Gln Arg Val Phe Thr Gly Ile Val Thr Ser
    50                  55                  60

Leu His Asp Tyr Phe Gly Val Val Asp Glu Glu Val Phe Phe Gln Leu
65                  70                  75                  80

Ser Val Val Lys Gly Arg Leu Pro Gln Leu Gly Glu Lys Val Leu Val
                85                  90                  95

Lys Ala Ala Tyr Asn Pro Gly Gln Ala Val Pro Trp Asn Ala Val Lys
            100                 105                 110

Val Gln Thr Leu Ser Asn Gln Pro Leu Leu Lys Ser Pro Ala Pro Pro
        115                 120                 125

Leu Leu His Val Ala Ala Leu Gly Gln Lys Gln Gly Ile Leu Gly Ala
    130                 135                 140

Gln Pro Gln Leu Ile Phe Gln Pro His Arg Ile Pro Pro Leu Phe Pro
145                 150                 155                 160

Gln Lys Pro Leu Ser Leu Phe Gln Thr Ser His Thr Leu His Leu Ser
                165                 170                 175

His Leu Asn Arg Phe Pro Ala Arg Gly Pro His Gly Arg Leu Asp Gln
            180                 185                 190

Gly Arg Ser Asp Asp Tyr Asp Ser Lys Lys Arg Lys Gln Arg Ala Gly
        195                 200                 205

Gly Glu Pro Trp Gly Ala Lys Lys Pro Arg His Asp Leu Ser Pro Tyr
    210                 215                 220

Arg Val His Leu Thr Pro Tyr Thr Val Asp Ser Pro Thr Cys Asp Phe
225                 230                 235                 240

Leu Glu Leu Gln Arg Arg Tyr Arg Ser Leu Leu Val Pro Ser Asp Phe
                245                 250                 255

Leu Ser Val His Leu Ser Trp Leu Ser Ala Phe Pro Leu Gly Gln Pro
            260                 265                 270

Phe Ser Leu His His Pro Ser Arg Ile Gln Val Ser Ser Glu Lys Glu
        275                 280                 285

Ala Ala Pro Asp Thr Gly Ala Glu Pro Ser Pro Glu Asp Ser Asp Pro

290                 295                 300

Thr Tyr Ser Ser Lys Val Leu Leu Leu Ser Ser Pro Gly Leu Glu Glu
305                 310                 315                 320

Phe Tyr Arg Cys Cys Met Leu Phe Val Asp Asp Met Ala Glu Pro Arg
                325                 330                 335

Glu Thr Pro Glu His Pro Leu Lys Gln Leu Lys Phe Leu Leu Gly Arg
            340                 345                 350

Lys Glu Glu Glu Ala Val Leu Val Gly Gly Glu Trp Ser Pro Ser Leu
        355                 360                 365

Asp Gly Leu Asp Pro Gln Ala Asp Pro Gln Val Leu Val Arg Thr Ala
    370                 375                 380

Ile Arg Cys Ala Gln Ala Gln Thr Gly Ile Asp Leu Ser Thr Cys Thr
385                 390                 395                 400

Lys Trp Trp Arg Phe Ala Glu Phe Gln Tyr Leu Gln Pro Gly Pro Pro
                405                 410                 415

Arg Gln Leu His Thr Val Val Val Tyr Leu Pro Asp Val Trp Thr Ile
            420                 425                 430

Met Pro Thr Leu Glu Glu Trp Glu Ala Leu Cys Gln Gln Lys Ala Thr
        435                 440                 445

Glu Ala Ala Pro Gln Pro His Glu Ala Ser Gly Glu Ala Glu Ala Thr
    450                 455                 460

Glu Gln Ala Pro Asp Val Ser Glu Gln Ala Asp Thr Ser Lys Gln Asn
465                 470                 475                 480

Thr Glu Thr Met Glu Ala Thr Thr Gln Gln Asp Val Asp Thr Asp Leu
                485                 490                 495

Pro Glu Ala Pro Pro Pro Pro Leu Glu Pro Ala Val Met Ala Arg Pro
            500                 505                 510

Arg Cys Val Asn Leu Ser Leu Tyr Gly Ile Val Glu Asp Arg Arg Pro
        515                 520                 525

Lys Glu Arg Ile Ser Phe Glu Val Val Val Leu Ala Glu Leu Phe Val
    530                 535                 540

Glu Met Leu Gln Arg Asp Phe Gly Tyr Arg Ile Tyr Lys Thr Leu Leu
545                 550                 555                 560

Ser Leu Pro Glu Lys Val Val Ser Pro Pro Glu Pro Glu Lys Glu Glu
                565                 570                 575

Ala Ala Lys Glu Asp Ala Val Lys Glu Glu Glu Ala Val Lys Glu Glu
            580                 585                 590

Ala Val Lys Val Ser Lys Asp Glu Val Gln Asn Glu Gly Thr Ala Ala
        595                 600                 605

Glu Ser Asp Ser Pro Leu Lys Glu Asp Gly Leu Leu Pro Lys Arg Pro
    610                 615                 620

Ser Ser Gly Gly Glu Glu Glu Glu Lys Ala Arg Gly Glu Ala Ala Glu
625                 630                 635                 640

Asp Leu Cys Glu Met Ala Leu Asp Pro Asp Leu Leu Leu Leu Arg Asp
                645                 650                 655

Asp Gly Glu Asp Glu Phe Ala Gly Ala Lys Leu Glu Glu Thr Glu Val
            660                 665                 670

Arg Ser Val Ala Ser Asn Gln Ser Glu Met Glu Tyr Ser Ser Leu Gln
        675                 680                 685

Asp Met Pro Lys Glu Leu Asp Pro Ser Thr Val Leu Pro Leu Asp Cys
    690                 695                 700

Leu Leu Ala Phe Val Phe Phe Asp Ala Asn Trp Cys Gly Tyr Leu His
705                 710                 715                 720

Arg Arg Asp Leu Glu Arg Val Leu Leu Thr Leu Gly Ile Arg Leu Ser
                725                 730                 735

Ala Glu Gln Ala Lys Gln Leu Val Ser Arg Val Val Ala Gln Asn Ile
            740                 745                 750

Cys Gln Tyr Arg Ser Leu Gln Tyr Ser Arg Ala Glu Val Leu Asp Asp
        755                 760                 765

Gly Leu Pro Glu Asp Val Leu Phe Gly Asn Leu Asp Leu Leu Pro Pro
    770                 775                 780

Ser Gly Lys Ser Thr Lys Pro Gly Ala Ala Pro Thr Glu His Lys Gly
785                 790                 795                 800

Leu Val Pro His Asn Gly Ser Leu Ile Asn Val Gly Ser Leu Leu Gln
                805                 810                 815

Arg Ala Glu Gln Gln Asp Ser Gly Arg Leu Tyr Leu Glu Asn Lys Ile
            820                 825                 830

His Thr Leu Glu Leu Lys Leu Glu Glu Ser His Asn Arg Phe Ser Ala
        835                 840                 845

Thr Glu Val Thr Asn Lys Thr Leu Ala Ala Glu Met Gln Glu Leu Arg
    850                 855                 860

Ala Arg Leu Ala Glu Ala Glu Glu Thr Ala Arg Thr Ala Glu Arg Gln
865                 870                 875                 880

Lys Asn Gln Leu Gln Arg Gln Met Gln Asp Phe Arg Arg Arg Leu Thr
                885                 890                 895

Pro Leu His Leu Glu Met Gln Arg Ile Val Glu Lys Ala Asp Ser Trp
            900                 905                 910

Val Glu Lys Glu Glu Pro Thr Pro Ser Asn
        915                 920

<210>  5
<211>  21
<212>  DNA
<213>  artificial sequence

<220>
<223>  DNA target sequence for siRNA inhibiting human Dbc1

<400>  5
tagtcatgca agctggtaac a                                                21

<210>  6
<211>  471
<212>  DNA
<213>  mus musculus

<220>
<221>  CDS
<222>  (50)..(337)

<220>
<221>  sig_peptide
<222>  (50)..(118)

<220>
<221>  proprotein
<222>  (119)..(334)

<220>
<221>  polyA_signal
<222>  (454)..(459)

<220>
<221>  polyA_site
<222>  (471)..(471)

<400>  6
agaacagaca agtcccacac agcagcttgg tgcacaccta gcagacacc atg agg acc      58
                                                      Met Arg Thr
                                                      1

ctc tct ctg ctc act ctg ctg gcc ctg gct gcg ctc tgt ctc tct gac      106
Leu Ser Leu Leu Thr Leu Leu Ala Leu Ala Ala Leu Cys Leu Ser Asp
    5                   10                  15

ctc aca gat gcc aag ccc agc ggc cct gag tct gac aaa gcc ttc atg      154

Leu Thr Asp Ala Lys Pro Ser Gly Pro Glu Ser Asp Lys Ala Phe Met
20                  25                  30                  35

tcc aag cag gag ggc aat aag gta gtg aac aga ctc cgg cgc tac ctt          202
Ser Lys Gln Glu Gly Asn Lys Val Val Asn Arg Leu Arg Arg Tyr Leu
                40                  45                  50

gga gcc tca gtc ccc agc cca gat ccc ctg gag ccc acc cgg gag cag          250
Gly Ala Ser Val Pro Ser Pro Asp Pro Leu Glu Pro Thr Arg Glu Gln
            55                  60                  65

tgt gag ctt aac cct gct tgt gac gag cta tca gac cag tat ggc ttg          298
Cys Glu Leu Asn Pro Ala Cys Asp Glu Leu Ser Asp Gln Tyr Gly Leu
        70                  75                  80

aag acc gcc tac aaa cgc atc tac ggt atc act att tag gacctgtgct          347
Lys Thr Ala Tyr Lys Arg Ile Tyr Gly Ile Thr Ile
    85                  90                  95

gccctaaagc caaactctgg cagctcggct ttggctgctc tccgggactt gatcctccct          407

gtcctctctc tctgccctgc aagtatggat gtcacagcag ctccaaaata aagttcagat          467

gagg                                                                      471

<210>  7
<211>  95
<212>  PRT
<213>  mus musculus

<400>  7

Met Arg Thr Leu Ser Leu Leu Thr Leu Leu Ala Leu Ala Ala Leu Cys
1               5                   10                  15

Leu Ser Asp Leu Thr Asp Ala Lys Pro Ser Gly Pro Glu Ser Asp Lys
            20                  25                  30

Ala Phe Met Ser Lys Gln Glu Gly Asn Lys Val Val Asn Arg Leu Arg
        35                  40                  45

Arg Tyr Leu Gly Ala Ser Val Pro Ser Pro Asp Pro Leu Glu Pro Thr
    50                  55                  60

Arg Glu Gln Cys Glu Leu Asn Pro Ala Cys Asp Glu Leu Ser Asp Gln
65                  70                  75                  80

Tyr Gly Leu Lys Thr Ala Tyr Lys Arg Ile Tyr Gly Ile Thr Ile
                85                  90                  95

<210>  8
<211>  46
<212>  PRT
<213>  mus musculus

<400>  8

Tyr Leu Gly Ala Ser Val Pro Ser Pro Asp Pro Leu Glu Pro Thr Arg
1               5                   10                  15

Glu Gln Cys Glu Leu Asn Pro Ala Cys Asp Glu Leu Ser Asp Gln Tyr

20                  25                  30

Gly Leu Lys Thr Ala Tyr Lys Arg Ile Tyr Gly Ile Thr Ile
        35                  40                  45

<210>  9
<211>  552
<212>  DNA
<213>  homo sapiens

<220>
<221>  CDS
<222>  (73)..(375)

<220>
<221>  sig_peptide
<222>  (73)..(141)

<220>
<221>  proprotein
<222>  (142)..(372)

<220>
<221>  polyA_signal
<222>  (511)..(516)

<220>
<221>  polyA_site
<222>  (534)..(534)

<400>  9
aacagtgctg gaggctggcg gggcaggcca gctgagtcct gagcagcagc ccagcgcagc      60

caccgagaca cc atg aga gcc ctc aca ctc ctc gcc cta ttg gcc ctg gcc     111
              Met Arg Ala Leu Thr Leu Leu Ala Leu Leu Ala Leu Ala
              1               5                   10

gca ctt tgc atc gct ggc cag gca ggt gcg aag ccc agc ggt gca gag       159
Ala Leu Cys Ile Ala Gly Gln Ala Gly Ala Lys Pro Ser Gly Ala Glu
    15                  20                  25

tcc agc aaa ggt gca gcc ttt gtg tcc aag cag gag ggc agc gag gta       207
Ser Ser Lys Gly Ala Ala Phe Val Ser Lys Gln Glu Gly Ser Glu Val
30                  35                  40                  45

gtg aag aga ccc agg cgc tac ctg tat caa tgg ctg gga gcc cca gtc       255
Val Lys Arg Pro Arg Arg Tyr Leu Tyr Gln Trp Leu Gly Ala Pro Val
                50                  55                  60

ccc tac ccg gat ccc ctg gag ccc agg agg gag gtg tgt gag ctc aat       303
Pro Tyr Pro Asp Pro Leu Glu Pro Arg Arg Glu Val Cys Glu Leu Asn
            65                  70                  75

ccg gac tgt gac gag ttg gct gac cac atc ggc ttt cag gag gcc tat       351
Pro Asp Cys Asp Glu Leu Ala Asp His Ile Gly Phe Gln Glu Ala Tyr
        80                  85                  90

cgg cgc ttc tac ggc ccg gtc tag ggtgtcgctc tgctggcctg gccggcaacc      405
Arg Arg Phe Tyr Gly Pro Val
    95                  100

ccagttctgc tcctctccag gcacccttct ttcctcttcc ccttgccctt gccctgacct     465

cccagcccta tggatgtggg gtccccatca tcccagctgc tcccaaataa actccagaag     525

aggaatctga aaaaaaaaaa aaaaaaa                                         552

<210>  10
<211>  100
<212>  PRT
<213>  homo sapiens

<400>  10

Met Arg Ala Leu Thr Leu Leu Ala Leu Leu Ala Leu Ala Ala Leu Cys
1               5                   10                  15

Ile Ala Gly Gln Ala Gly Ala Lys Pro Ser Gly Ala Glu Ser Ser Lys
            20                  25                  30

Gly Ala Ala Phe Val Ser Lys Gln Glu Gly Ser Glu Val Val Lys Arg
        35                  40                  45

Pro Arg Arg Tyr Leu Tyr Gln Trp Leu Gly Ala Pro Val Pro Tyr Pro
    50                  55                  60

Asp Pro Leu Glu Pro Arg Arg Glu Val Cys Glu Leu Asn Pro Asp Cys
65                  70                  75                  80

Asp Glu Leu Ala Asp His Ile Gly Phe Gln Glu Ala Tyr Arg Arg Phe
                85                  90                  95

Tyr Gly Pro Val
            100


<210>  11
<211>  49
<212>  PRT
<213>  homo sapiens

<400>  11

Tyr Leu Tyr Gln Trp Leu Gly Ala Pro Val Pro Tyr Pro Asp Pro Leu
1               5                   10                  15

Glu Pro Arg Arg Glu Val Cys Glu Leu Asn Pro Asp Cys Asp Glu Leu
            20                  25                  30

Ala Asp His Ile Gly Phe Gln Glu Ala Tyr Arg Arg Phe Tyr Gly Pro
        35                  40                  45

Val


<210>  12
<211>  862
<212>  DNA
<213>  artificial sequence

<220>
<223>  Promoter of human osteocalcin gene

<400>  12

ctgcagggtc aggaggagaa tcgtggggcc aggagggcag aggcacactc catcttcgtg 60
ctcctcacag gccctgcctc cctgcctgct aaggacacag ggaagggggt ccccacctca 120
gtgcctgcct cccttccctg tgcctgtgta cctggcagtc acagccacct ggcgtgtccc 180
agaaaccaac cggctgacct catctcctgc ccggccccac ctccattggc tttggctttt 240
ggcgtttgtg ctgcccgacc ctttctcctg tccggatgcg cagggcaggg cctgagccgt 300
cgagctgcac ccacagcagg ctgcctttgg tgactcaccg ggtgaacggg ggcattgcga 360
ggcatcccct ccctgggttt ggctcctgcc cacggggctg acagtagaaa tcacaggctg 420
tgagacagct ggagcccagc tctgcttgaa cctattttag gtctctgatc cccgcttcct 480
ctttagactc ccctagagct cagccagtgc tcaacctgag gctgggggtc tctgaggaag 540
agtgagttgg agctgagggg tctggggctg tcccctgaga gaggggccag aggcagtgtc 600
aagagccggg cagtctgatt gtggctcacc ctccatcact cccaggggcc cctggcccag 660
cagccgcagc tcccaaccac aatatccttt ggggtttggc ctacggagct ggggcggatg 720
acccccaaat agccctggca gattccccct agacccgccc gcaccatggt caggcatgcc 780
cctcctcatc gctgggcaca gcccagaggg tataaacagt gctggaggct ggcggggcag 840
gccagctgag tcctgagcag ca 862